(19)

Europäisches
Patentamt

European
Patent Office

Office européen
des brevets

(11) **EP 2 298 336 A2**

(12) **EUROPEAN PATENT APPLICATION**

(43) Date of publication:
   **23.03.2011 Bulletin 2011/12**

(51) Int Cl.:
   *A61K 38/18* (2006.01)      *A61L 27/54* (2006.01)
   *A61L 27/60* (2006.01)      *A61L 26/00* (2006.01)

(21) Application number: **10182861.4**

(22) Date of filing: **12.07.2006**

(84) Designated Contracting States:
   **AT BE BG CH CY CZ DE DK EE ES FI FR GB GR
   HU IE IS IT LI LT LU LV MC NL PL PT RO SE SI
   SK TR**

(30) Priority: **12.07.2005 GB 0514262**

(62) Document number(s) of the earlier application(s) in
   accordance with Art. 76 EPC:
   **06764933.5 / 1 904 089**

(71) Applicant: **Renovo Limited
   Manchester M13 9XX (GB)**

(72) Inventors:
   • **Ferguson, Mark William James
      Manchester M13 9XX (GB)**
   • **O'Kane, Sharon
      Manchester M13 9XX (GB)**

• **Occleston, Nicholas
   Manchester M13 9XX (GB)**
• **Laverty, Hugh
   Manchester M13 9XX (GB)**

(74) Representative: **Harrison Goddard Foote
   4th Floor
   Merchant Exchange
   17-19 Whitworth Street West
   Manchester
   M1 5WG (GB)**

Remarks:
   •Claims filed after the date of filing of the application
   / after the date of receipt of the divisional application
   (Rule 68(4) EPC).
   •This application was filed on 29-09-2010 as a
   divisional application to the application mentioned
   under INID code 62.

(54) **Promotion of epithelial regeneration**

(57)   There is provided an agent having TGF-$\beta_3$ activity for use in the promotion of epithelial regeneration. Suitable agents may be selected from the group consisting of TGF-$\beta_3$, a fragment, derivative or variant of TGF-$\beta_3$, a substance able to promote and/or mimic the biological activity of TGF-$\beta_3$. The agents may be used in a formulation that further comprises a sugar, such as a sugar selected from the group consisting of: monosaccharides and disaccharides, such as maltose, mannose, sucrose, glucose, or trehalose. Also provided are methods of preparing therapeutic formulations suitable for localised parenteral administration, for use in the promotion of epithelial regeneration; agents having TGF-$\beta_3$ activity for use preparing an epithelial graft donor site for re-harvesting; and delivery systems for use in gene therapy techniques.

EP 2 298 336 A2

SEQUENCE LISTING

<110> Renovo Limited

<120> Promotion of epithelial regeneration

<130> P119982EP1

<140> EP 10182861.4
<141> 2006-07-12

<150> GB0514262
<151> 2005-07-12

<160> 2

<170> PatentIn version 3.5

<210> 1
<211> 112
<212> PRT
<213> Homo sapiens

<400> 1

Ala Leu Asp Thr Asn Tyr Cys Phe Arg Asn Leu Glu Glu Asn Cys Cys
1               5                   10                  15

Val Arg Pro Leu Tyr Ile Asp Phe Arg Gln Asp Leu Gly Trp Lys Trp
            20                  25                  30

Val His Glu Pro Lys Gly Tyr Tyr Ala Asn Phe Cys Ser Gly Pro Cys
            35                  40                  45

Pro Tyr Leu Arg Ser Ala Asp Thr Thr His Ser Thr Val Leu Gly Leu
        50                  55                  60

Tyr Asn Thr Leu Asn Pro Glu Ala Ser Ala Ser Pro Cys Cys Val Pro
65                  70                  75                  80

Gln Asp Leu Glu Pro Leu Thr Ile Leu Tyr Tyr Val Gly Arg Thr Pro
                85                  90                  95

Lys Val Glu Gln Leu Ser Asn Met Val Val Lys Ser Cys Lys Cys Ser
                100                 105                 110

<210> 2
<211> 412
<212> PRT
<213> Homo sapiens

<400> 2

Met Lys Met His Leu Gln Arg Ala Leu Val Val Leu Ala Leu Leu Asn
1               5                   10                  15

Phe Ala Thr Val Ser Leu Ser Leu Ser Thr Cys Thr Thr Leu Asp Phe
            20                  25                  30

Gly His Ile Lys Lys Lys Arg Val Glu Ala Ile Arg Gly Gln Ile Leu
        35              40              45

Ser Lys Leu Arg Leu Thr Ser Pro Pro Glu Pro Thr Val Met Thr His
        50              55              60

Val Pro Tyr Gln Val Leu Ala Leu Tyr Asn Ser Thr Arg Glu Leu Leu
65              70              75              80

Glu Glu Met His Gly Glu Arg Glu Glu Gly Cys Thr Gln Glu Asn Thr
                85              90              95

Glu Ser Glu Tyr Tyr Ala Lys Glu Ile His Lys Phe Asp Met Ile Gln
            100             105             110

Gly Leu Ala Glu His Asn Glu Leu Ala Val Cys Pro Lys Gly Ile Thr
        115             120             125

Ser Lys Val Phe Arg Phe Asn Val Ser Ser Val Glu Lys Asn Arg Thr
    130             135             140

Asn Leu Phe Arg Ala Glu Phe Arg Val Leu Arg Val Pro Asn Pro Ser
145             150             155             160

Ser Lys Arg Asn Glu Gln Arg Ile Glu Leu Phe Gln Ile Leu Arg Pro
                165             170             175

Asp Glu His Ile Ala Lys Gln Arg Tyr Ile Gly Gly Lys Asn Leu Pro
            180             185             190

Thr Arg Gly Thr Ala Glu Trp Leu Ser Phe Asp Val Thr Asp Thr Val
        195             200             205

Arg Glu Trp Leu Leu Arg Arg Glu Ser Asn Leu Gly Leu Glu Ile Ser
    210             215             220

Ile His Cys Pro Cys His Thr Phe Gln Pro Asn Gly Asp Ile Leu Glu
225             230             235             240

Asn Ile His Glu Val Met Glu Ile Lys Phe Lys Gly Val Asp Asn Glu
            245             250             255

Asp Asp His Gly Arg Gly Asp Leu Gly Arg Leu Lys Lys Gln Lys Asp
            260             265             270

His His Asn Pro His Leu Ile Leu Met Met Ile Pro Pro His Arg Leu
        275             280             285

Asp Asn Pro Gly Gln Gly Gly Gln Arg Lys Lys Arg Ala Leu Asp Thr
        290             295             300

```
Asn Tyr Cys Phe Arg Asn Leu Glu Glu Asn Cys Cys Val Arg Pro Leu
305             310             315                 320

Tyr Ile Asp Phe Arg Gln Asp Leu Gly Trp Lys Trp Val His Glu Pro
            325             330                 335

Lys Gly Tyr Tyr Ala Asn Phe Cys Ser Gly Pro Cys Pro Tyr Leu Arg
            340             345                 350

Ser Ala Asp Thr Thr His Ser Thr Val Leu Gly Leu Tyr Asn Thr Leu
            355             360             365

Asn Pro Glu Ala Ser Ala Ser Pro Cys Cys Val Pro Gln Asp Leu Glu
    370             375             380

Pro Leu Thr Ile Leu Tyr Tyr Val Gly Arg Thr Pro Lys Val Glu Gln
385             390             395                 400

Leu Ser Asn Met Val Val Lys Ser Cys Lys Cys Ser
            405             410
```

**Description**

The present invention relates to the manufacture of medicaments for the promotion of epithelial regeneration. It also provides a method of promoting epithelial regeneration in an individual in need of such promotion.

The body comprises many different types of epithelia, the structures and complexity of which vary depending on their location, role, and function.

The most basic form of epithelium is the simple epithelium, which comprises a single layer of epithelial cells. An example of a form of simple epithelium is simple squamous epithelium, which comprises a single layer of flattened scale-like cells. Examples of simple squamous epithelia in the body include mesothelium, endothelium, and the lining of pulmonary alveoli.

Another common form of epithelium is the stratified epithelium. This comprises a series of layers, and the cells of different layers may vary in size, shape and function. Stratified epithelia may be further characterised with reference to the type of cells located at their surface.

By way of example, stratified squamous epithelia have scale-like (squamous) cells located at their surface. The shape of the cells comprising stratified squamous epithelia may vary with different locations within the epithelium. Whilst cells located at the upper surface of the epithelium generally have a flattened scale-like conformation, those towards the base of the epithelium may tend to have a polyhedral shape. Epithelial cells may be structurally fortified by the presence of keratin, and the quantity of this molecule present tends to increase with increasing height within the epithelia. Stratified squamous epithelia typically comprise several layers of keratin-containing cells. The types of keratin molecule present may vary by both cell layer and body site.

The most prominent of the body's epithelia is the epidermis, the stratified squamous epithelial layer that covers the skin. The epidermis, by virtue of its location, is the tissue most frequently in contact with the external environment, and as a result is the tissue most frequently exposed to environmental, and other, damage.

Further examples of stratified epithelia include stratified columnar epithelia, and stratified ciliated columnar epithelia, in which the uppermost layer of cells are columnar in shape, and provided with motile cilia.

Epithelia throughout the body are subject to many different forms of damage. Such damage may impair or entirely destroy the function of the epithelia injured, and the outcome of such damage depends on the nature and role of the epithelium affected. Accordingly the promotion of epithelial regeneration is advantageous in many different contexts. However, despite the desirability of the promotion of epithelial regeneration, there remains a requirement for further, and more effective, medicaments and methods by which such promoted regeneration may be attained.

There is significant variation in the range of therapies currently used to promote epithelial regeneration. For example, the management of split thickness skin graft donor sites (as reviewed in Rakel *et al.* 1998) may merely involve leaving the graft donor site exposed and untreated, or may alternatively make use of treatments such as the application of dressings (typically gauze dressings, which may be used alone or impregnated with a variety of anti-infective agents, alginates, hydrocolloids, synthetic composite membranes, transparent films or honey), application of artificial skin (which may be generated from the individuals own epidermis), application of allografts (typically bovine or porcine allografts) or application of ointments (typically ointments containing silver based compounds as anti-infective agents).

This absence of a single universally accepted therapy is indicative of the need for novel methods by which epithelial regeneration may be promoted. Furthermore, it is well recognised that there are many failings and disadvantages associated with current therapies.

The adverse effects associated with current treatments include protracted healing times at the graft donor and/or recipient sites, the development of replacement epithelium that may be rougher and/or thinner than that originally present, increased rates of infection, edema and erythema, hypertrophic scarring around the treatment site, risk of prion or viral infection from allograft material, the length of time taken to generate the treatment (in the case of artificial skin generation from the patients own cells) and pain associated with both donor and recipient sites.

It is an object of the present invention to provide new medicaments for the promotion of epithelial regeneration that overcome at least some of the disadvantages associated with the prior art.

According to a first aspect of the present invention there is provided the use of an agent having TGF-$\beta_3$ activity in the manufacture of a medicament for the promotion of epithelial regeneration.

The agent having TGF-$\beta_3$ activity may preferably be selected from the group comprising TGF-$\beta_3$, biologically active fragments, variants and derivatives of TGF-$\beta_3$, and substances able to promote and/or mimic the biological activity of TGF-$\beta_3$.

The promotion of epithelial regeneration within the context of the present invention may be understood to encompass any increase in the rate of epithelial regeneration as compared to the regeneration occurring in a control-treated or untreated epithelium.

The rate of epithelial regeneration attained by methods in accordance with the invention may readily be compared with that taking place in control-treated or untreated epithelia using any suitable model of epithelial regeneration known in the art. For example, the rate at which sites of experimental epithelial damage having known areas regenerate may be compared using well *known in vivo* models in mice, rats, rabbits or pigs such as those described in Tomlinson and Ferguson (2003), Davidson *et al.* (1991) and Paddock *et al.* (2003).

In accordance with a second aspect of the invention there is provided a method of promoting epithelial regeneration, the method comprising administering a therapeutically effective amount of an agent having TGF-$\beta_3$ activity to a subject in need of such promotion.

In relation to this aspect of the invention a "therapeutically effective amount of an agent having TGF-$\beta_3$ activity" is an amount of an agent, having TGF-$\beta_3$ activity, that is sufficient to promote epithelial regeneration in the subject to whom the amount is administered.

As noted above, epithelia, and particularly the epidermis, suffer more direct, frequent, and damaging encounters with the external environment than any other tissue in the body. It is therefore highly desirable that the repair and renewal of epithelial tissues, such as the epidermis, be able to be influenced in order to ensure their maximum functional effectiveness. Indeed, although the methods and medicaments of the invention are suitable for the promotion of epithelial regeneration in all types of epithelia, and have been effective in all epithelia tested, the promotion of epidermal regeneration constitutes a particularly preferred application of the invention. Other preferred embodiments of the invention include the promotion of squamous epithelial regeneration, and/or the promotion of keratinised epithelial regeneration. It will be appreciated that the methods and medicaments of the invention may be of benefit to the epidermis covering the scalp.

The promotion of epithelial regeneration in accordance with the invention is able to bring about the formation of a functioning epithelial barrier over previously damaged or denuded areas. The epithelial barrier formed is able to prevent ingress into, and colonisation of, the underlying tissue by pathogens such as bacteria, fungi and viruses. Thus the promotion of epithelial regeneration may provide benefits in contexts in which it is desirable to prevent or reduce infection at sites where the epithelial layer has been breached.

The presence of an intact epithelial layer also acts as a barrier to fluid movement, and is therefore able to prevent desiccation of underlying tissue. Thus promotion of epithelial regeneration is able to prevent or reduce tissue desiccation arising as a result of fluid loss across a damaged or otherwise breached epithelial layer.

The present invention is based on the very surprising finding that epithelial regeneration may be stimulated by the provision of the cytokine TGF-$\beta_3$, or an agent that shares the characteristic biological activity of TGF-$\beta_3$. TGF-$\beta$s (which exist in three mammalian isoforms, TGF-$\beta_1$, TGF-$\beta_2$ and TGF-$\beta_3$) have previously been thought to inhibit epithelial regeneration.

The inhibitory effects of TGF-$\beta$ on epithelial regeneration have been reported based on *both in vitro* and *in vivo* assays. Studies of the effects of TGF-$\beta$ isoforms on cultured keratinocytes (cutaneous epithelial cells) have suggested that, although TGF-$\beta$ treatment increases keratinocytes' expression of integrins associated with migration, the rate of keratinocyte proliferation, which is necessary for epidermal regeneration, is considerably reduced. TGF-$\beta$-mediated inhibition of epithelial cell proliferation required for epithelial regeneration has also been reported using *in vivo* studies.

Although the reported inhibitory effects of TGF-$\beta$ are exhibited by all isoforms there are differences in the relative potencies of the different isoforms. Studies have shown that TGF-$\beta_3$ is the most potent of the isoforms in its ability to inhibit DNA synthesis and proliferation in primary cultures of human keratinocytes, bringing about stronger inhibition than do either TGF-$\beta_1$ or TGF-$\beta_2$.

In addition to studies using cultured cells, a number of *in vivo* studies have recently been undertaken, utilising specific compounds or genetic technologies to disrupt the TGF-$\beta$ signalling pathway. These studies have further suggested that TGF-$\beta$s, and particularly TGF-$\beta_3$, inhibit epithelial regeneration.

In contrast to the previously published reports, the inventors of the present application have now discovered that the application of an agent having TGF-$\beta_3$ activity to an epithelial tissue before or after damage to that tissue is able to promote the regeneration of the epithelial layer, rather than inhibiting such regeneration. Without wishing to be bound by any hypothesis the inventors believe that the promotion of epithelial regeneration is achieved by TGF-$\beta$-mediated promotion of epithelial cell migration. The epithelial cells (the migration of which has been promoted) are thereby able to re-populate and regenerate the damaged epithelium more rapidly than occurs in the absence of agents having TGF-$\beta_3$ activity.

It will be appreciated that promotion of epithelial regeneration in accordance with the invention may be of use to induce effective re-epithelialisation in contexts in which invention may be of benefit in instances where the re-epithelialisation response is impaired, inhibited, retarded or otherwise defective. However, it is particularly preferred that the methods and medicaments of the invention are use in the promotion of epithelial regeneration may be also effected to accelerate the rate of normal epithelial regeneration responses in patients suffering from epithelial damage.

There are many contexts in which the body's re-epithelialisation response may be defective. For example, defective re-epithelialisation in the skin is associated with conditions such as pemphigus, Hailey-Hailey disease (familial benign pemphigus), toxic epidermal necrolysis (TEN)/Lyell's syndrome, epidermolysis bullosa, cutaneous leishmaniasis and actinic keratosis. Defective re-epithelialisation of the lungs may be associated with idiopathic pulmonary fibrosis (IPF) or interstitial lung disease. Defective re-epithelialisation of the eye may be associated with conditions such as partial limbal stem cell deficiency or corneal erosions. Defective re-epithelialisation of the gastrointestinal tract or colon may be associated with conditions such as chronic anal fissures (fissure in ano), ulcerative colitis or Crohn's disease, and other inflammatory bowel disorders.

The process of re-epithelialisation in response to dermal injuries may also be perturbed in many individuals. For example, it is well known that dermal injuries in the aged exhibit less-vigorous epithelial regeneration than do those of younger individuals. There are also many other conditions or disorders that are associated with delayed or otherwise impaired epithelial regeneration in response to injury. For example patients with diabetes, patients with polypharmacy (for example as a result of old age), post-menopausal women, patients susceptible to pressure injuries (for example paraplegics), patients with venous disease, clinically obese patients, patients receiving chemotherapy, patients receiving radiotherapy, patients receiving steroid treatment or immuno-compromised patients may all suffer from impaired epithelial regeneration. In many such cases the lack of a proper epithelial regeneration response contributes to the development of infections at the wound site, and to retardation of the normal wound healing response that may lead to the formation of chronic wounds such as ulcers. Accordingly, it will be appreciated that epithelial promotion by the methods or medicaments of the invention will be of benefit to such patients.

Without detracting from the above, it may generally be preferred that promotion of epithelial regeneration in accordance with the invention may be effected in order to augment an ongoing re-epithelialisation response (i.e. to produce a greater maximal epithelial regeneration response than would normally be achieved without promotion). It will be appreciated that in this way sites of epithelial damage occurring in otherwise healthy subjects may be induced to regenerate more rapidly.

Much experimental and research effort has been expended on the identification and development of therapeutic agents and techniques that may be used to promote epithelial regeneration in those for whom the re-epithelialisation response is impaired. However, the group of patients who do not suffer from impaired re-epithelialisation constitutes a greater number of the total population covered by the healthcare services, and a far larger proportion of the working populace. The skilled person will immediately appreciate, therefore, that there is a great benefit to be gained by society from the development of therapeutic agents and techniques that can address the needs of these otherwise healthy patients. Accordingly, the promotion of epithelial regeneration in healthy patients is a preferred embodiment of all aspects of the present invention. The promotion of re-epithelialisation of acute wounds (as opposed to chronic wounds) is also a preferred embodiment of all aspects of the invention.

For the purposes of the present invention, a chronic wound may be defined as any wound that does not show any healing tendency within eight weeks of formation when subject to appropriate (conventional) therapeutic treatment. Acute wounds may be any wound other than a chronic wound. Preferred acute wounds may be incisional wounds, of which surgical incisional wounds may be a particularly preferred group.

Epithelia, such as the epidermis or corneal epithelium, may be subject to damage as a result of many different types of insult. Epithelia may, for example, be injured as a result of physical insults or injuries, which include grazes, abrasions, wounds (both penetrating wounds and non-penetrating wounds), surgical incisions, and other surgical procedures (particularly partial thickness grafts of tissues such as the skin), "burns" (which, except for where the context requires otherwise, may be considered to include tissue damage resulting from exposure to either high or low temperature, chemical agents or radiation), and other forms of trauma.

The inventors have found that the medicaments and methods of the invention are particularly effective in the promotion of epithelial regeneration in response to injuries. These are exemplified, a particularly of benefit to, injuries to the skin, in which the epidermis is damaged. It will however be appreciated that the methods of the invention may also be applicable to other types of injury and wounds including injury, damage or trauma to epithelia such as the respiratory epithelia, or those surrounding internal tissues or organs. The epithelium regeneration of which is to be promoted may be an epithelium other than a digestive epithelium. For instance the epithelium may be other than the intestinal or gastrointestinal epithelium.

It is recognised that epithelial damage, and particularly epidermal damage, resulting from burns may extend over great areas of an individual so afflicted. As a result burn injuries are particularly susceptible to complications such as infection and desiccation that arise due to lack of a functional epithelial layer. Accordingly, the promotion of epithelial regeneration in response to burn damage represents a preferred application of the invention.

The promotion of epithelial regeneration, for instance at the site of dermal injuries, brings about a rapid improvement in the cosmetic appearance of the injured area. Cosmetic considerations are important in a number of clinical contexts, particularly when epithelial damage occurs at prominent body sites such as the face, neck and hands. Consequently the promotion of epithelial regeneration at sites where it is desired to improve the cosmetic appearance of the damaged area represents a preferred embodiment of the invention.

Damage to epithelia may also arise as a result of the action of pathogens (such as bacteria, fungi, or viruses), chemical insults (such as chemical burns caused by caustic agents, or through the effect of cytotoxic drugs such as those employed in chemotherapy) or as a result of radiation damage (either through particulate radiation or electromagnetic radiation such as gamma radiation, ultraviolet radiation, or the like) such as that occurring in sunburn. Promotion of epithelial regeneration using methods and medicaments in accordance with the invention may be utilised effectively in all of the above-mentioned contexts.

It will be appreciated that many of the same considerations that arise in relation to epithelial damage in humans can also be problematic in other animals, particularly veterinary or domestic animals (e.g. horses, cattle, dogs, cats etc). Thus the methods of the invention may also be applicable to non-human animals.

The use of agents having TGF-$\beta_3$ activity to promote epithelial regeneration in response to damage associated with epithelial grafting procedures represents a preferred embodiment of the invention. Epithelial damage occurring as a result of skin grafts can be clinically problematic, in addition to cosmetic considerations.

Epithelial regeneration is of benefit both at the epithelial graft donor site, where it aids in the re-establishment of a functional epithelial layer, and also at the recipient site where regeneration is able to improve and accelerate integration of the grafted tissue. Epithelial regeneration at graft recipient sites is also advantageous in the case of grafts utilising skin, artificial skin, or skin substitutes.

Promotion of epithelial regeneration at epithelial graft donor sites decreases the time taken to restore a functioning epithelial layer, and consequently reduces the potential for donor site infection. The promotion of epithelial regeneration also decreases incidences of blistering and tissue breakdown that may otherwise occur at the donor site.

It is preferred that the epithelial grafts are epidermal (skin) grafts. The methods and medicaments of the invention have utility in promoting epithelial regeneration in the contexts of both full and partial thickness skin grafts. Such skin grafts (i.e. either full or partial thickness grafts) may be either meshed or unmeshed.

The inventors have found that the presence or absence of an intact epithelial layer is also a factor in determining the degree of pain associated with sites at which the epithelium has been damaged or removed, such as skin donor sites. Thus by promoting epithelial regeneration at such sites it is possible to reduce the pain associated with, for example, the taking of skin grafts.

A further advantage of promotion of epithelial regeneration at epithelial donor sites is that this decreases the time required until re-harvesting of tissue from the donor site can take place. By re-harvesting is meant the subsequent removal of further epithelial tissue from a previously used donor site. This is particularly advantageous in situations where the skin available for harvesting is limited and/or the area of skin required to be harvested is large. Examples of such situations include occasions when it is necessary to take grafts from children and/or patients suffering from burns covering a large percentage of the body surface.

Accordingly, in a third aspect of the invention there is provided a method of preparing an epithelial graft donor site for re-harvesting, the method comprising administering to an epithelial donor site in need of such preparation a therapeutically effective amount of an agent having TGF-$\beta_3$ activity. In the context of this aspect of the invention a "therapeutically effective amount" is an amount of an agent having TGF-$\beta_3$ activity sufficient to promote epithelial regeneration to such an extent that further epithelial grafts may be taken from the donor site. It will be appreciated that the decision as to whether a donor site is sufficiently regenerated to allow re-harvesting will normally be one undertaken by a competent physician, and that such a person will have access to a wealth of guidance, both by way of relevant texts and their own experience, to indicate how soon such re-harvesting may normally be undertaken without the benefit of preparation in accordance with the invention.

The inventors have found that the promotion of epithelial regeneration brought about by agents having TGF-$\beta_3$ activity may be effected using TGF-$\beta_3$ itself, biologically active fragments, variants and derivatives of TGF-$\beta_3$, and substances able to promote or mimic the biological activity of TGF-$\beta_3$. Suitable biologically active fragments, variants and derivatives of TGF-$\beta_3$ may readily be identified by their ability to replicate the effects of TGF-$\beta_3$ *in vivo* or *in vitro*. TGF-$\beta_3$ activity may be assessed *in vivo* using suitable animal models in which the rate of epithelial regeneration occurring in areas of epithelial damage treated with test compounds may be compared with the rate of epithelial regeneration occurring in control areas of epithelial damage. Suitable animal models may include partial thickness or full thickness wounds of tissues containing an epithelial layer (such as the epidermis of skin).

The skilled person will appreciate that when assessing the rate of epithelial regeneration (for example, in order to identify whether or not regeneration has been promoted) the relevant parameter to be considered is the extent of epithelial coverage achieved. Care should be taken to differentiate such epithelial coverage from other factors, such as the amount of granulation tissue (or other materials such as matrix associated with clot formation) present in a wound. Granulation tissue of this sort may contribute to wound closure, by filling a wound void or the bed of a wound, but does not contribute to epithelial regeneration, since it is essentially composed of non-epithelial cell types. In the case of analysis of wounds (such as skin wounds) epithelial cells may typically be identified by virtue of their flattened morphology (as opposed to "spindle" shaped fibroblasts, or rounded cells of the inflammatory response).

The amino acid sequence of native human TGF-$\beta_3$ is provided as Sequence ID No. 1 below:

ALDTNYCFRN LEENCCVRPL YIDFRQDLGW

KWVHEPKGYY ANFCSGPCPY LRSADTTHST

VLGLYNTLNP EASASPCCVP QDLEPLTILY

YVGRTPKVEQ LSNMVVKSCK CS

(Sequence ID No. 1)

The prior art contains a number of variant forms of TGF-$\beta_3$ that are suitable for use in the methods and medicaments of the invention. For example, EP-A-0 684 260 describes a polypeptide comprising a modified form of TGF-$\beta_3$ having suitable biological activity for use in accordance with the present invention, as well as nucleic acids encoding the same. The disclosures of EP-A-0 684 260 represent preferred polypeptides and nucleic acids for use in accordance with the present invention.

Variants of TGF-$\beta_3$ that may be used in accordance with the invention include proteins containing conserved amino acid substitutions that retain the biological activity of TGF-$\beta_3$ as characterised by its ability to promote epithelial regeneration. It is preferred that conserved substitutions may be substitutions designed to remove protease cleavage sites, or other peptide structures that may be involved in the degradation or clearance of TGF-$\beta_3$. Further details of variants and derivatives of TGF-$\beta_3$ that may be employed in the medicaments and methods of the invention are provided below.

Suitable variant forms of TGF-$\beta_3$ may be ones in which certain of the native amino acids are replaced with amino acids having a side chain of similar biophysical properties to the amino acid it substitutes, to produce a conservative change. For example small non-polar, hydrophobic amino acids include glycine, alanine, leucine, isoleucine, valine, proline, and methionine. Large non-polar, hydrophobic amino acids include phenylalanine, tryptophan and tyrosine. The polar neutral amino acids include serine, threonine, cysteine, asparagine and glutamine. The positively charged (basic) amino acids include lysine, arginine and histidine. The negatively charged (acidic) amino acids include aspartic acid and glutamic acid.

Other modifications in protein sequences such as those which occur during or after translation, e.g. by acetylation, amidation, carboxylation, phosphorylation, proteolytic cleavage or linkage to a ligand may provide further variant forms of TGF-$\beta_3$ suitable for use in the medicaments and methods of the invention.

Derivatives of TGF-$\beta_3$ suitable for use in the methods and medicaments of the invention may include derivatives that increase or decrease the TGF-$\beta_3$'s half-life in vivo. Examples of derivatives capable of increasing the half-life of TGF-$\beta_3$ include peptoid derivatives of TGF-$\beta_3$, D-amino acid derivatives of TGF-$\beta_3$, and peptide-peptoid hybrids.

It will be appreciated that, since TGF-$\beta_3$ and its fragments (as well as many of the possible variants and derivatives thereof) are proteins or may contain peptidyl components, they may be subject to degradation by a number of means (such as protease activity in biological systems). Such degradation may limit the bioavailability of the polypeptides and hence the ability of the polypeptides to achieve their biological function. There are wide ranges of well-established techniques by which peptide derivatives that have enhanced stability in biological contexts can be designed and produced. Such peptide derivatives may have improved bioavailability as a result of increased resistance to protease-mediated degradation. Preferably, a peptide derivative or analogue suitable for use according to the invention is more protease-resistant than the peptide from which it is derived.

Preferably, TGF-$\beta_3$, its variants or fragments, may be made more protease-resistant by protecting the N and/or C terminal. For example, the N terminal may be protected by an acetyl group. The C terminal may be protected by an amide group.

Protease-resistance of a derivative of TGF-$\beta_3$ may be compared with protease-resistance of TGF-$\beta_3$ itself by means of well-known protein degradation assays described in the prior art.

Peptoid derivatives of TGF-$\beta_3$ may be readily designed from knowledge of TGF-$\beta_3$'s structure. Commercially available software may be used to develop peptoid derivatives according to well-established protocols.

Retropeptoids, (in which all amino acids are replaced by peptoid residues in reversed order) are also able to mimic the epithelial regeneration promoting properties of TGF-$\beta_3$. A retropeptoid is expected to bind in the opposite direction in the ligand-binding groove, as compared to a peptide or peptoid-peptide hybrid containing one peptoid residue. As a result, the side chains of the peptoid residues are able to point in the same direction as the side chains in the original peptide.

D-amino acid forms of TGF-$\beta_3$ constitute a further embodiment of a derivative of TGF-$\beta_3$ suitable for use in accordance with the methods and medicaments of the invention. In this case, the order of the amino acid residues comprising the derivative is reversed as compared to the original TGF-$\beta_3$. The preparation of derivatives using D-amino acids rather than L-amino acids greatly decreases any unwanted breakdown of such an agent by normal metabolic processes, decreasing the amounts of agent which need to be administered, along with the frequency of its administration.

It will be appreciated that, in order to constitute a biologically active fragment, variant, or derivative of TGF-$\beta_3$ suitable for use in accordance with the methods and medicaments of the invention a fragment, variant, or derivative must retain the epithelial regeneration promoting activity of TGF-$\beta_3$.

TGF-$\beta_3$ (or biologically active fragments, variants, or derivatives thereof) may be provided for use in the methods or medicaments of the invention in an active or inactive form. TGF-$\beta_3$ may be inactivated by any of a number of mechanisms, for example, by encapsulation. Capsules may be degradable by an external stimulus to release the active agent when required. External stimuli suitable for use in this manner include UV light, ultrasound, *in vivo* enzymes or heat.

TGF-$\beta_3$ may also be provided as an inactive precursor, which may be activated upon contact with tissue containing the natural cleavage enzymes required to convert the precursor into its active form. The amino acid sequence of a naturally occurring precursor of human TGF$\beta_3$ is provided as Sequence ID No. 2 below:

| | | |
|---|---|---|
| MKMHLQRALV | VLALLNFATV | SLSLSTCTTL |
| DFGHIKKKRV | EAIRGQILSK | LRLTSPPEPT |
| VMTHVPYQVL | ALYNSTRELL | EEMHGEREEG |
| CTQENTESEY | YAKEIHKFDM | IQGLAEHNEL |
| AVCPKGITSK | VFRFNVSSVE | KNRTNLFRAE |
| FRVLRVPNPS | SKRNEQRIEL | FQILRPDEHI |
| AKQRYIGGKN | LPTRGTAEWL | SFDVTDTVRE |
| WLLRRESNLG | LEISIHCPCH | TFQPNGDILE |
| NIHEVMEIKF | KGVDNEDDHG | RGDLGRLKKQ |
| KDHHNPHLIL | MMIPPHRLDN | PGQGGQRKKR |
| ALDTNYCFRN | LEENCCVRPL | YIDFRQDLGW |
| KWVHEPKGYY | ANFCSGPCPY | LRSADTTHST |
| VLGLYNTLNP | EASASPCCVP | QDLEPLTILY |
| YVGRTPKVEQ | LSNMVVKSCK | CS |

## (Sequence ID No. 2)

Inactivation may, alternatively be achieved by the molecular addition of a binding molecule. The binding molecule may be detachable when required by an external stimulus such as UV light, ultrasound, *in vivo* enzymes or heat.

Suitable peptides for use in the production of inactive TGF-$\beta_3$ are well known in the art, since TGF-$\beta$s are often secreted from cells in an inactive form known as latent TGF-$\beta$. Latent TGF-$\beta$ consists of an N terminal Latency Associated Peptide (LAP) and the TGF-$\beta$ and is also referred to as the Small Latent Complex. Additionally the Small Latent Complex can bind to another peptide (derived from a different gene) of variable size called Latent TGF-$\beta$ Binding Protein (LTBP) in which case the entire complex is known as the Large Latent TGF-$\beta$ Complex.

Latent TGF-$\beta$ is activated when the TGF-$\beta$ is caused to be dissociated from the LAP. This dissociation may be co-ordinated at a mannose-6-phosphate / Insulin Like Growth Factor II receptor (M6P-R) and involve proteases such as plasmin, the substrates being associated at the cell surface by tissue transglutaminase. Free radicals and reactive oxygen species can also activate TGF-$\beta$ by causing dissociation from the LAP.

The invention also encompasses the use of substances capable of promoting or mimicking the biological activity of TGF-$\beta_3$ for the manufacture of a medicament for promoting epithelial regeneration.

Substances capable of promoting or mimicking the biological activity of TGF-$\beta_3$ may achieve their effect by a number of means. For instance, such substances may increase the expression of TGF-$\beta_3$, or they may increase the half-life of TGF-$\beta_3$, for example by decreasing turnover of TGF-$\beta_3$. Examples of substances capable of promoting or mimicking the biological activity TGF-$\beta_3$ include both proteinaceous and non-proteinaceous substances. For example, such substances capable of promoting or mimicking the biological activity of TGF-$\beta_3$ include transcription factors regulating TGF-$\beta_3$ activity, activating antibodies capable of mimicking the biological activity of TGF-$\beta_3$, small inorganic molecules that replicate the receptor-binding effects of TGF-$\beta_3$, as well as substances able to induce intracellular signaling cascades that retain the characteristics of those generated on receptor binding of TGF-$\beta_3$.

Preferably the promotion of epithelial regeneration may give rise to a rate of epithelial regeneration that is at least 5%, 10%, 20% or 30% greater than the regeneration occurring in a control-treated or untreated epithelium. More preferably promoted epithelial regeneration may give rise to a rate of epithelial regeneration that is at least 40%, 50% or 60% greater than regeneration occurring in a control-treated or untreated epithelium. It is even more preferred that promoted epithelial regeneration may give rise to a rate of epithelial regeneration that is at least 70%, 80%, or 90% greater than regeneration occurring in a control-treated or untreated epithelium, and most preferably promoted epithelial regeneration may give rise to a rate of epithelial regeneration that is at least 100% greater than regeneration occurring in a control-treated or untreated epithelium.

Preferably the promotion of epithelial regeneration may give rise to a time to re-epithelialise 1 day, 2 days, or 3 days faster than that occurring in a control-treated or untreated epithelium. More preferably promoted epithelial regeneration may give rise to a time to re-epithelialise that is at least 4 days, 5 days or 6 days faster than that occurring in a control-treated or untreated epithelium. It is even more preferred that promoted epithelial regeneration may give rise to a time to re-epithelialise that is at least 7 days, 8 days or 9 days faster than that occurring in a control-treated or untreated epithelium, and most preferably promoted epithelial regeneration may give rise to a time to re-epithelialise that is at least 10 days or greater than that occurring in a control-treated or untreated epithelium.

With respect to the time to re-harvesting of epithelium, for example from donor sites for split thickness skin grafts, preferably the promotion of epithelial regeneration may give rise to a time to re-harvesting that is 1 day, 2 days, or 3 days faster than that occurring in a control-treated or untreated epithelium. More preferably promoted epithelial regeneration may give rise to a time to re-harvesting that is at least 4 days, 5 days or 6 days faster than that occurring in a control-treated or untreated epithelium. It is even more preferred that promoted epithelial regeneration may give rise to a time to re-harvesting that is at least 7 days, 8 days or 9 days faster than that occurring in a control-treated or untreated epithelium, and most preferably promoted epithelial regeneration may give rise to a time to re-harvesting that is at least 10 days or greater than that occurring in a control-treated or untreated epithelium.

Medicaments in accordance with the invention may provide therapeutically effective amounts of agents in accordance with the invention suitable for promoting epithelial regeneration. The inventors have found that such medicaments are able to promote epithelial regeneration when administered either prior to epithelial damage, or once such damage has already occurred.

The prophylactic use of agents in accordance with the invention to promote epithelial regeneration is a preferred mode of use in accordance with the invention. It will be appreciated that such use is most suitable in the case where the time and location of prospective epithelial damage is known, for example damage occurring as a result of elective procedures, but pre-treatment with agents in accordance with the invention is also considered in situations where there is a likelihood of epithelial damage arising. The inventors have found that administration of agents in accordance with the invention immediately prior to epithelial damage (i.e. in the hour, or preferably half hour, preceding the occurrence of damage) is highly effective, though administration at earlier times (e.g. up to 24 or 48 hours before epithelial damage) is also effective. The prophylactic use of uses, methods and medicaments in accordance with the invention is a preferred embodiment of the invention, and is particularly preferred in the preparation of skin graft donor and/or recipient sites.

Agents in accordance with the invention are also effective in promoting epithelial regeneration when administered after epithelial damage has occurred. It is preferred that such administration should occur as early as possible after the initiation of damage, but agents in accordance with the invention are able to promote epithelial regeneration at any time up until full restoration of the damaged epithelium has taken place. It will be appreciated that the "window" in which agents in accordance with the invention may be effectively administered such that they are able to promote epithelial regeneration is dependent on a number of factors, including the nature of the epithelium in question (including the epithelium's natural rate of repair), the degree of damage that has occurred, and the size of the damaged area. Thus in the case of a large area of epithelial damage, or in the case of damage to an epithelium that is naturally slow to regenerate, agents in accordance with the invention may be still be effectively administered relatively late in the regeneration response. Agents in accordance with the invention may, for instance, preferably be administered within the first one to 24 hours after epithelial damage has occurred, but may still achieve beneficial promotion of epithelial regeneration if administered up to ten, or more, days after the initiation of damage.

Promotion of epithelial regeneration may be achieved by repeated administration of agents in accordance with the invention at sites of epithelial damage. For instance therapeutically effective amounts of agents in accordance with the invention may be administered to damaged epithelia as required until full epithelial regeneration has been achieved.

By way of example agents in accordance with the invention may be administered daily or twice daily to a site of epithelial damage for at least the first three days following the occurrence of the damage.

Most preferably agents in accordance with the invention are administered both before and after incidences of epithelial damage. The inventors have found that administration of agents in accordance with the invention immediately prior to epithelial damage, followed by daily administration of such agents for the three days following epithelial damage, is particularly effective in promoting epithelial regeneration.

It will be appreciated that the amount of an agent in accordance with the invention to be applied to a site of epithelial damage depends on a number of factors such as the biological activity and bioavailability of the agent, which in turn depends on the mode of administration and the physicochemical properties of the agent. Other factors may include:

A) The half-life of the agent in the subject being treated.
B) The specific condition to be treated.
C) The age of the subject.

The frequency of administration will also be influenced by the above mentioned factors and particularly the half-life of the chosen agent in accordance with the invention within the subject being treated.

Generally when agents in accordance with the invention are used to treat existing sites of epithelial damage the agent should be administered as soon as the epithelial damage has occurred or has been diagnosed. Therapy with agents in accordance with the invention should continue until the damaged epithelium has regenerated to a clinician's satisfaction.

Frequency of administration will depend upon the biological half-life of the agent in accordance with the invention used. Typically a cream or ointment containing an agent in accordance with the invention should be administered to a target tissue such that the concentration of the agent at the site of epithelial damage is maintained at a level suitable for having a therapeutic effect. This may require administration daily or even several times daily.

Therapeutically effective amounts of agents in accordance with the invention, for instance in the form of medicaments in accordance with the invention, may be administered by any suitable route capable of achieving the desired effect of promoting epithelial regeneration, but may preferably be administered locally at sites of epithelial damage.

The inventors have found that epithelial regeneration may be effectively promoted by the administration of an agent in accordance with the invention (particularly TGF-$\beta_3$) in the form of injections at sites of epithelial damage. For instance, in the case of damage to the epidermis, the agent in accordance with the invention may be administered by means of intradermal injection. Thus a preferred composition of the invention comprises an injectable solution of an agent in accordance with the invention (e.g. for injection around the margins of a site of epithelial damage or a site likely to be damaged). Suitable formulations for use in this embodiment of the invention are considered in the Appendix below.

Alternatively, or additionally, an agent in accordance with the invention may also be administered in a topical form to promote epithelial regeneration. Such administration may be effected as part of the initial and/or follow up care for the damaged area. Details of formulations suitable for topical administration are set out later. Topical formulations may be applied by injection onto the wound surface, by aerosol spray and also by application onto the surface of the wound under an occlusive or semi-occlusive dressing e.g., Opsite, Bioclusive, Tegaderm or the like.

The inventors find that the promotion of epithelial regeneration in accordance with the present invention is particularly improved by topical application of an agent in accordance with the invention to damaged epithelia (or, in the case of prophylactic application, to epithelia that are to be damaged).

It is preferred that compositions containing agents in accordance with the invention are formulated such that they additionally comprise a sugar. The sugar may preferably be selected from the group comprising maltose, mannose, sucrose and glucose. It is particularly preferred that the sugar is maltose. The sugar, such as maltose, may be present in sufficient quantity that the composition is substantially isotonic having regard to the site at which the composition is to be administered.

The inventors have found that the use of sugars such as maltose in formulations provides a marked and surprising improvement in terms of both the amount of active material that can be recovered from vessels containing TGF-$\beta_3$ (e.g., pre-filled syringes) and the inherent biological activity of the TGF-$\beta_3$ molecule. With respect to the recovery of TGF-$\beta_3$ material, formulation in maltose resulted in a 4-fold increase in recoverable material (as measured by ELISA) compared to a mannitol-based formulation. With respect to the inherent biological activity of TGF-$\beta_3$, formulation in maltose resulted in a 4-fold increase in activity ($IC_{50}$ in the Mink Lung Epithelial Cell assay of 7.309 [$\pm$1.044] pg/mL) compared to the mannitol formulation (IC50 in the Mink Lung Epithelial Cell assay of 30.104 [$\pm$7.093] pg/mL).

The use of compositions in which agents in accordance with the invention are formulated in the presence of sugars such as maltose are particularly preferred in contexts where the compositions are to be administered by means of injection. The inventors have surprisingly found that the use of sugars such as maltose in such formulations provides notable advantages in terms of reduced levels of pain experienced by those receiving injections of the compositions, as compared to formulations in which other tonicity buffering agents (such as mannitol) are used.

Indeed, so beneficial are compositions comprising active agents in accordance with the invention and sugars such as maltose that they constitute a separate aspect of the invention. Accordingly, in an fourth aspect of the invention there is provided a composition comprising an agent having TGF-$\beta_3$ activity formulated in the presence of a sugar selected from the group comprising maltose, mannose, sucrose and glucose. It is preferred that the sugar comprises maltose.

Suitable compositions for use in accordance with any of the preceding aspects of the invention may comprise active agents formulated in the presence of a sugar such as maltose at a concentration of between 0.1M and 0.4M of the sugar. More preferably suitable formulations may comprise the active agents formulated in the presence of 0.25M sugar.

Compositions comprising active agents in accordance with the invention may take a number of different forms depending, in particular, on the manner in which they are to be used. Thus, for example, they may be in the form of a liquid, ointment, cream, gel, hydrogel, powder or aerosol. All of such compositions are suitable for topical application to a damaged epithelium, which is a preferred means of administering agents in accordance with the invention to a subject (person or animal) in need of treatment.

In a preferred embodiment therapeutic formulations of the agents of the invention suitable for localised parenteral administration (e.g. intradermal and sub-cutaneous) may be prepared by mixing the agent (having the desired degree of purity) with optional physiologically acceptable carriers, excipients or stabilizers in the form of lyophilised and non-lyophilised powder formulations for reconstitution, non-aqueous and aqueous solutions, non-aqueous and aqueous dispersions/suspensions including emulsions and semi-solid formulations. Acceptable carriers, including excipients, are non-toxic to recipients at the dosages and concentrations employed, and include, but are not limited to, buffers such as phosphates, citrates, and other organic acids; antioxidants including ascorbic acid and methionine; tonicity modifiers such as sodium chloride, glucose, glycerol, and alike; preservatives such as octadecyldimethylbenzyl ammonium chloride; hexamethonium chloride; benazalkonium chloride, benzethonium chloride; phenol, butyl or benzyl alcohol; alkyl parabens such as methyl and/or propyl and/or butyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; other sugars such as sucrose, mannitol, maltose, trehalose or sorbitol; salt-forming counter-ions such as sodium; metal complexes (e.g., Zn-protein complexes); anionic surfactants such as fatty acid soaps, acyl sulphates, or acyl sulfosuccinates; cationic surfactants, such as alkyl primary, secondary, tertiary, or quaternary amines; non-ionic surfactants, for example, sorbitan esters or polyethoxylated esters of acyl acids, copolymers of polyethylene oxide and polypropylene oxide.

A pharmaceutical formulation example of a sterile solution for localised parenteral administration of the present invention, in addition to the active agents, may optionally or additionally include the following:

0.01 M to 0.1 M phosphate buffer, and

Sodium chloride up to 0.9% w/v (to achieve iso-tonicity with blood, 290-300 mOsm/L))

1 to 10 w/v % maltose (although another suitable sugar may be used in the alternative), and

0.1 mg/ml polyoxyethylene sorbitan mono-oleate (Tween™ 80).

It will be readily appreciated by the skilled person that a lyophilised (freeze-dried) powder 'cake' may be prepared based on the above solution.

Preferred embodiments of the invention may be presented in the form of a vial, an ampoule, or a pre-filled syringe of either; a sterile solution; a sterile lyophilised (freeze-dried) powder suitable for reconstitution; a sterile suspension or any other pharmaceutically acceptable form of presentation suited to localised parenteral drug delivery.

In a further preferred embodiment of the invention therapeutic formulations of the agents of the invention suitable for topical administration may be prepared by mixing the substance having the desired degree of purity with optional physiologically acceptable carriers, excipients or stabilisers in the form of lyophilised or non-lyophilised powder formulations, non-aqueous or aqueous solutions, non-aqueous or aqueous dispersions/suspensions, including emulsions and semi-solid formulations. Acceptable carriers, including excipients, are non-toxic to recipients at the dosages and concentrations employed, and include, but are not limited to, purified water, saline, phosphate-buffered saline (PBS) Ringer's solution, Ringer's-lactate solution, dextrose solutions, dextrose/saline solution, hydro-alcoholic solutions, glucose, sucrose, dextran, mannose, mannitol, maltose, sorbitol, polyethylene glycol (PEG), propylene glycol (PG), phosphates, acetates, gelatin, collagens, Carbopol 934™ (BF Goodrich Corp.), vegetable and synthetic oils and waxes, anionic surfactants such as fatty acid soaps, acyl sulfates, or acyl sulfosuccinates; cationic surfactants, such as alkyl primary, secondary, tertiary, or quaternary amines; non-ionic surfactants, for example, sorbitan esters or polyethoxylated esters of acyl acids, copolymers of polyethylene oxide and polypropylene oxide, and the like. One may additionally include suitable preservatives, stabilisers, antioxidants, anti-microbials and buffering agents, for example, methyl and/or propyl and/or butyl parabens, butylated hydroxy anisole (BHA), butylated hydroxy toluene (BHT), citric acid, ascorbic acid, and the like. Emulsion, cream or ointment bases useful in formulation may include aqueous-based creams and emulsions (oil-in-water), oil-based creams and emulsions (water-in-oil), ointments (emulsifying and non-emulsifying hydrocarbon), gels, hydrogels, and the like. Other formulations for topical delivery may include aerosols, bandages, and other wound dressings. Alternatively one may incorporate or encapsulate the therapeutic compound of the invention in a suitable polymer matrix or membrane, thus providing a sustained-release delivery device suitable for placement on, or implantation near, the site to be treated.

A pharmaceutical formulation example of a semi-solid hydrogel formulation for topical administration of the present invention, in addition to the active ingredient/s, may include the following:

0.1 % w/v to 2.0% w/v hydroxy cellulose, and

0.1 % w/v to 1.0% w/v Carbopol 934™ (BF Goodrich Corp.), and

10 to 20% w/v propylene glycol, and

0.005% w/v to 0.020% w/v methyl paraben, and

0.005% w/v to 0.020% w/v propyl paraben, and

Sodium hydroxide or hydrochloric acid q.s. ad pH 4 -10

Purified water, q.s. ad 100% w/v

Suitable compositions for topical application in accordance with the invention (including those compositions in which active agents in accordance with the invention are formulated in the presence of maltose) may be presented in the form of a bottle, a jar, a tube, a spray, of, either; a sterile solution; a sterile lyophilised (freeze-dried) or non-lyophilised powder for reconstitution, a sterile dispersion/suspension, a sterile semi-solid, or any other pharmaceutically acceptable form of presentation suited to topical drug delivery.

The agents in accordance with the invention may be provided on a sterile dressing or patch, which may be used to cover a site of epithelial damage to be treated.

It will be appreciated that the vehicle of the composition comprising agents in accordance with the invention should be one which is well tolerated by the patient and allows release of the agent to the site of epithelial damage. Such a vehicle is preferably biocompatible, biodegradeable, bioresorbable, bioresolveable and/or non-inflammatory.

Compositions comprising agents in accordance with the invention may be used in a number of ways. Thus, for example, a composition may be applied in and/or around a site of epithelial damage to regulate epithelial regeneration. If the composition is to be applied to an "existing" site of epithelial damage, then the pharmaceutically acceptable vehicle will be one which is relatively "mild" i.e. a vehicle which is biocompatible, biodegradable, bioresolvable and non-inflammatory.

An agent in accordance with the invention, or a nucleic acid encoding such an agent, may be incorporated within a slow or delayed release device. Such devices may, for example, be placed on or inserted under the skin and the agent or nucleic acid may be released over days, weeks or even months. Such a device may be particularly useful for patients requiring long-term promotion of epithelial regeneration. The devices may be particularly advantageous when used for the administration of an agent or nucleic acid which would normally require frequent administration (e.g. at least daily administration by other routes).

Daily doses of an agent in accordance with the invention may be given as a single administration (e.g. a daily application of a topical formulation or a daily injection). Alternatively, the agent in accordance with the invention may require administration twice or more times during a day. In a further alternative, a slow release device may be used to provide optimal doses of an agent in accordance with the invention to a patient without the need to administer repeated doses.

In one embodiment a pharmaceutical vehicle for administration of an agent in accordance with the invention may be a liquid and a suitable pharmaceutical composition would be in the form of a solution. In another embodiment, the pharmaceutically acceptable vehicle is a solid and a suitable composition is in the form of a powder or tablet. In a further embodiment the agent in accordance with the invention may be formulated as a part of a pharmaceutically acceptable transdermal patch.

A solid vehicle can include one or more substances which may also act as flavoring agents, lubricants, solubilizers, suspending agents, fillers, glidants, compression aids, binders or tablet-disintegrating agents; it can also be an encapsulating material. In powders, the vehicle is a finely divided solid which is in admixture with the finely divided agent in accordance with the invention. In tablets, the agent in accordance with the invention is mixed with a vehicle having the necessary compression properties in suitable proportions and compacted in the shape and size desired. The powders and tablets preferably contain up to 99% of the agent in accordance with the invention. Suitable solid vehicles include, for example, calcium phosphate, magnesium stearate, talc, sugars, lactose, dextrin, starch, gelatin, cellulose, polyvinylpyrrolidine, low melting waxes and ion exchange resins.

Liquid vehicles may be used in preparing solutions, suspensions, emulsions, syrups, elixirs and pressurized compositions. The agent in accordance with the invention can be dissolved or suspended in a pharmaceutically acceptable liquid vehicle such as water, an organic solvent, a mixture of both or pharmaceutically acceptable oils or fats. The liquid vehicle can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid vehicles for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the vehicle can also be an oily ester such as ethyl oleate and isopropyl myristate. Sterile liquid vehicles are useful in sterile liquid form compositions for parenteral administration. The liquid vehicle for pressurized compositions can be a halogenated hydrocarbon or other pharmaceutically acceptable propellant.

Liquid pharmaceutical compositions Which are sterile solutions or suspensions can be utilized by for example, intramuscular, intrathecal, epidural, intraperitoneal, intradermal or subcutaneous injection. Sterile solutions can also be administered intravenously and topically by aerosol spray or application below an occlusive or semi occlusive dressing. The agent in accordance with the invention may be prepared as a sterile solid composition e.g. by freeze drying or lyophilisation which may be dissolved or suspended at the time of administration using sterile water, saline, or other appropriate sterile injectable medium. Vehicles are intended to include necessary and inert binders, suspending agents, lubricants, flavorants, sweeteners, preservatives, dyes, and coatings.

In the situation in which it is desired to administer an agent in accordance with the invention by means of oral ingestion, it will be appreciated that the chosen agent will preferably be an agent having an elevated degree of resistance to degradation. For example, the agent in accordance with the invention may preferably not be a peptide, or may not have peptide components.

Compositions of agents in accordance with the invention are suitable to be used for reducing or controlling damage to the corneal epithelium (cells overlying the stromal cells of the cornea). Such damage may result from trauma to the eye arising as a result of accidental injury (as considered above) or as a result of surgical operations (e.g. laser surgery on the cornea). In this case the composition or medicament in accordance with the invention may be in the form of an eye drop. Although the compositions may be used in the cornea, the inventors believe that the methods of the invention may preferably be used in tissues, such as the skin or scalp, without stromal cells.

Agents in accordance with the invention may be used in a range of "internal" sites of epithelial damage (i.e. those sites of epithelial damage occurring within the body, rather than on an external surface). Thus for example, medicaments comprising agents in accordance with the invention may be formulated for inhalation for use in sites of epithelial damage arising in the lungs or other respiratory epithelia.

Known procedures, such as those conventionally employed by the pharmaceutical industry (e.g. *in vivo* experimentation, clinical trials etc), may be used to establish specific formulations of compositions comprising agents in accordance with the invention and precise therapeutic regimes for administration of such compositions (such as daily doses of the active agent and the frequency of administration).

Generally, compositions comprising agents in accordance with the invention should be formulated such that when administered to a site of epithelial damage a concentration of the agent of between of between 0.01nM and 10mM per $cm^2$ or linear cm is achieved at the site. Preferably, compositions comprising agents in accordance with the invention should be formulated such that when administered to a site of epithelial damage a concentration of the agent between 0.1nM and 1mM per $cm^2$ or linear cm is achieved at the site. More preferably, compositions comprising agents in accordance with the invention should be formulated such that when administered to a site of epithelial damage a concentration of the agent between 0.1nM and 400μM per $cm^2$ or linear cm is achieved at the site.

Purely by way of example an injectable solution containing between 50ng/100μl and 500ng/100μl of an agent in accordance with the invention (such as TGF-$\beta_3$) is suitable for application to a site of partial thickness epidermal damage.

A suitable daily dose of an agent in accordance with the invention able to promote epithelial regeneration depends upon the factors discussed above as well as upon the size of the site of epithelial damage to be treated. Typically the amount of an agent in accordance with the invention required for the treatment of site of epithelial damage will be within the range of 0.01nM to 10mM of the agent per $cm^2$ or linear cm per 24 hours, depending upon the area of the site of epithelial damage amongst several other factors.

Agents in accordance with the invention may be used to promote epithelial regeneration as a monotherapy (i.e. use of the agent alone). Alternatively the uses, methods or medicaments of the invention may be used in combination with other compounds or treatments able to promote epithelial regeneration. For example the uses, methods or medicaments of the invention may be used in combination with dressings (which may include gauzes, synthetic composite membranes and/or transparent films, any of which may optionally be impregnated with anti-infective agents, alginates, hydrocolloids or honey), artificial skin (such as artificial skin generated from an individual's own epidermis, or commercially available equivalents) or ointments (such as those comprising silver-based anti-infective compounds). Two examples of commercially available artificial skins suitable for use in combination with the methods, uses or medicaments of the invention are available under the names Apligraf (Graftskin) and Dermagraft.

The uses, methods or medicaments of the invention are also suitable for use in combination with other conventional or developmental therapies. The following paragraphs contemplate examples of such therapies, and make reference to the indications in which use of combination therapies may be preferred.

When promoting epithelial regeneration in the context of wound healing (and particularly the healing of burns and skin ulcers) it may be preferred to combine the uses, methods or medicaments of the invention with debriding agents. Suitable examples of such agents may include enzymic agents such as collagenase (Smith & Nephew), chemical agents, surgical/mechanical agents or biological agents e.g. maggot therapy.

Alternatively or additionally, when promoting epithelial regeneration in wound healing it may be preferred to combine the uses, methods or medicaments of the invention with lytic peptides such as cytoporins. These peptides may incorporate into biological cell membranes thereby causing lysis of the cells. An example of a suitable cytoporin for use in combination therapy is HB-107 (produced by Helix BioMedix, Inc.).

Epithelial regeneration in wound healing (particularly of diabetics), epidermolysis bullosa and ocular diseases (including corneal wound healing) may be promoted by the use of thymosin beta-4 in combination with the uses, methods and medicaments of the invention. A recombinant form of thymosin beta-4 suitable for such use is produced by RegeneRx Biopharmaceuticals Inc.

Another agent suitable for use in combination therapy with the uses, methods or medicaments of the invention for the promotion of epithelial regeneration associated with wound healing and disorders of the skin such as ulcers is Pimilprost. This compound is a stable analogue of Prostaglandin I1, and has biological activity as both an inhibitor of platelet aggregation and a vasodilator. Pimilprost suitable for use in combination with the claimed uses, methods and medicaments may be obtained from Sumitomo Pharmaceuticals Co. Ltd.

Epithelial regeneration in the oral cavity, for instance in the treatment of wounds or mouth ulcers, may be promoted by the combination of uses, methods and medicaments of the invention with mast cell degranulation inhibitors such as amlexanox (commercially available from Access Pharmaceuticals, Inc.).

Regeneration of epithelia damaged in wound healing or as a result of diabetic complications may also be promoted by the combination of uses methods and medicaments according to the present invention with heat shock protein-70 (HSP-70) or heat shock protein inducers, Bimoclomal, produced by Biorex Research and Development Co., represents a preferred example of a HSP inducer suitable for use in such combination therapy.

In order to promote epithelial regeneration in tissues such as the lining of the stomach (for example in the case of re-epithelialisation to promote healing of stomach ulcers), the epidermis, and corneal epithelium it may be preferred to combine the uses, methods and medicaments of the present invention with melanostatin analogues able to accelerate tissue regeneration. An example of a suitable analogue of this sort is Alaptid, which is available commercially from VUFB.

Another agent that may be advantageously used in combination with the uses, methods and medicaments of the invention is the enzyme heparinase III. Such combinations may preferably be used to promote epithelial regeneration associated with wound healing and the treatment of exterior ulcers (particularly diabetic ulcers). A suitable form of heparinase III for use in such combinations is produced by IBEX Technologies, Inc. under the designation IBT-9302.

The uses, methods and medicaments of the invention may additionally or alternatively be used in combination with the synthetic thrombin peptidomimetic Chrysalin produced by Chrysalis BioTechnology, Inc. Such combinations may be preferred for the promotion of epithelial regeneration associated with wound healing and diabetic ulcers, particularly diabetic foot ulcers.

The promotion of epithelial regeneration at sites of burn damage may be effected by the uses, methods or medicaments of the invention in combination with PV-707, manufactured by GroPep Ltd. PV-707 is a peptide growth factor agonist.

Promotion of epithelial regeneration in wounds, and particularly bums, may be promoted by the use of synthetic dehydroepiandrosterone sulphate (DHEAS) in combination with the uses, methods and medicaments of the invention. A form of synthetic DHEAS suitable for such use is manufactured by Pharmadigm Inc. under the name PB-005.

Epithelial regeneration in the treatment of dermatological disease and wound healing may be promoted by the uses, methods or medicaments of the invention in combination with recombinant lactoferrin. A suitable form of recombinant human lactoferrin is manufactured by Agennix Inc.

Another combination therapy suitable for the promotion of epithelial regeneration associated with the wound healing response lies in the combination of the uses, methods or medicaments of the invention with the provision of free deoxyribonucleosides. A suitable source of such deoxyribonucleosides is provided by PN-105, which comprises an equiweight mixture of these molecules in a gel base and is manufactured by Wellstat Therapeutics Corp.

There exists a range of methods and compositions designed to improve or augment epithelial regeneration that utilise growth factors other than TGF-$\beta_3$. It will be appreciated that the uses, methods or medicaments of the invention may also be used in combination with these existing treatments. The following paragraphs provide further guidance as to how the present invention may be used in combination with other growth factor-based treatments.

The uses, methods and medicaments of the invention may be used in combination with members of the fibroblast growth factor (FGF) family. For instance, the present invention may be used in combination with basic FGF (FGF-2). This combination of the uses, methods or medicaments of the invention with FGF-2 may be particularly preferred in the promotion of epithelial regeneration following wound damage. Examples of wound sites that may benefit from such a combination include burns, graft donor sites, and chronic wounds such as ulcers (including non-healing ulcers such as diabetic ulcers or decubiti). The FGF-2 may preferably be recombinant FGF-2 (rFGF-2) and more preferably recombinant human FGF-2 (rhFGF-2). A suitable example of rFGF-2 suitable for use in accordance with this embodiment of the invention is that produced by Scios Inc. or Chiron.

Another member of the fibroblast growth factor family that may be used in combination with the uses, methods and medicaments of the invention to promote epithelial regeneration is FGF-10 (also known as keratinocyte growth factor-2 or KGF-2) to promote the regeneration of epithelia damaged as a result of wounding, complications of the dermal healing process (such as skin ulcers), diseases such as oral mucositis or ulcerative colitis, or gastrointestinal epithelial damage such as that occurring in Crohn's disease. A suitable form of FGF-10 suitable for use in combination with the uses, methods or medicaments of the invention is produced by Human Genome Sciences, Inc. under the name Repifermin.

The uses, methods and medicaments of the invention may also be used in combination with members of the platelet derived growth factor (PDGF) family. For example, the combination of the uses, methods or medicaments of the invention with PDGF-B may be of use in promoting epithelial regeneration in wound healing, and particularly the healing of burns wounds or diabetic foot ulcers. In one preferred embodiment of such combinations the PDGF-B to be used may preferably be delivered by means of an adenoviral vector. Selective Genetics, Inc. produces a suitable example of such an adenoviral vector under the name AdPDGF-B/GAM.

Another suitable combination utilises the uses, methods or medicaments of the invention in combination with PDGF-BB. An example of PDGF-BB which may be used in such a combination is commercially available under the name Regranex.

The combination of the uses, methods or medicaments of the invention with cytokine inhibitors may also be used to promote epithelial regeneration. Such combinations may, for instance, be used in promoting epithelial regeneration in response to injury, or to treat epithelial damage occurring as a result of diseases such as irritable bowel disease (IBD) or Crohn's disease. A suitable example of such an inhibitor is Semapimod (CNI-1493) a synthetic guanylhydrazone MAPK inhibitor produced by the Picower Institute for Medical Research.

Agents according to the invention may alternatively or additionally be provided in combination with compounds able to inhibit protease activity. Protease inhibitors may be chosen on the basis of a broad spectrum of inhibitory activity, or for the ability to selectively inhibit proteases (or protease families) present at sites of epithelial damage. Proteases to be inhibited may include neutrophil elastase, matrix metalloproteinases, plasminogen activators (for example urokinase plasminogen activator or tissue plasminogen activator), plasmin, cathepsins, furin, and members of the "a disintegrin and a metalloproteinase" family such as ADAM or ADAM-TS. Suitable protease inhibitors may include peptide, protein, or small molecule inhibitors.

It will be appreciated that TGF-$\beta_3$, its fragments, derivatives and variants, as well as agents capable of increasing the biological activity of TGF-$\beta_3$ may represent favourable agents to be administered by techniques involving cellular expression of nucleic acid sequences encoding such molecules. Such methods of cellular expression are particularly suitable for medical use in which the therapeutic effects of the polypeptides, derivatives and analogues are required over a prolonged period, for example in contexts where it is desirable to augment over a period of time an otherwise defective epithelial regeneration response.

Many known methods of administering agents in accordance with the invention to a relevant damaged epithelial tissue have the disadvantage that it can be difficult to achieve sustained levels of the agent at the site of epithelial damage over the course of even a few days because many suitable agents may have short half-lives *in vivo.* The half-lives of the agents may be short for a number of reasons which include:

(i) Degradation by proteases and the like.
(ii) Clearance by binding proteins.
(iii) Binding and inhibition of agent activity by extracellular matrix molecules such as decorin and fibronectin.

Furthermore, agents used to treat sites of epithelial damage healing need to be administered in a suitable vehicle and are often provided as a composition comprising the active agent and the vehicle. As discussed further below, such vehicles are preferably non-inflammatory, biocompatible, bioresorbable and must not degrade or inactivate the agent (in storage or in use). However, it can often be difficult to provide a satisfactory vehicle for delivering agents to a tissue to be treated.

A convenient way in which these problems can be obviated or mitigated is to provide a therapeutically effective amount of an agent in accordance with the invention at a site of epithelial damage by means of gene therapy.

According to a fifth aspect of the present invention there is provided a delivery system for use in a gene therapy technique, said delivery system comprising a DNA molecule encoding for an agent in accordance with the invention, said DNA molecule being capable of being transcribed to lead to the expression of the chosen agent.

According to a sixth aspect of the present invention there is provided the use of a delivery system as defined in the preceding paragraph for use in the manufacture of a medicament for use in the promotion of epithelial regeneration.

According to a seventh aspect of the present invention there is provided a method of promoting epithelial regeneration, the method comprising administering to a patient in need of treatment a therapeutically effective amount of a delivery system as defined for the fifth aspect of the invention.

Due to the degeneracy of the genetic code, it is clear that nucleic acid sequences encoding agents suitable for use in accordance with the invention may be varied or changed without substantially affecting the sequence of the product encoded thereby, to provide a functional variant thereof. As noted above, an agent suitable for use in accordance with the invention must retain the epithelial regeneration promoting activity of TGF-$\beta_3$.

Suitable nucleotide encoding variants of TGF-$\beta_3$ include those having a sequence altered by the substitution of different codons that encode the same amino acid within the sequence, thus producing a silent change. Other suitable variants are those having homologous nucleotide sequences but comprising all, or portions of, sequence which are altered by the substitution of different codons that encode an amino acid with a side chain of similar biophysical properties to the amino acid it substitutes, to produce a conservative change. For example small non-polar, hydrophobic amino acids include glycine, alanine, leucine, isoleucine, valine, proline, and methionine. Large non-polar, hydrophobic amino acids include phenylalanine, tryptophan and tyrosine. The polar neutral amino acids include serine, threonine, cysteine, asparagine and glutamine. The positively charged (basic) amino acids include lysine, arginine and histidine. The negatively charged (acidic) amino acids include aspartic acid and glutamic acid.

The delivery systems according to the invention are highly suitable for achieving sustained levels of an agent in accordance with the invention at a site of epithelial damage over a longer period of time than is possible for most conventional delivery systems. Agents in accordance with the invention suitable for promoting epithelial regeneration may be continuously expressed from cells at the site of epithelial damage that have been transformed with the DNA molecule disclosed in the fifth aspect of the invention. Therefore, even if the agent in accordance with the invention has a very short half-life *in vivo,* therapeutically effective amounts of the agent may be continuously expressed from the treated tissue.

Furthermore, the delivery system of the invention may be used to provide the DNA molecule (and thereby the agent in accordance with the invention) without the need to use conventional pharmaceutical vehicles such as those required in ointments or creams that are contacted with the site of epithelial damage.

The delivery system of the present invention is such that the DNA molecule is capable of being expressed (when the delivery system is administered to a patient) to produce an agent in accordance with the invention which directly or indirectly has activity for promoting epithelial regeneration. By "directly" we mean that the product of gene expression *per se* has the required activity for promoting epithelial regeneration. By "indirectly" we mean that the product of gene expression undergoes or mediates (e.g. as an enzyme) at least one further reaction to provide an active agent effective for promoting epithelial regeneration.

The DNA molecule may be contained within a suitable vector to form a recombinant vector. The vector may for example be a plasmid, cosmid or phage. Such recombinant vectors are highly useful in the delivery systems of the invention for transforming cells with the DNA molecule.

Recombinant vectors may also include other functional elements. For instance, recombinant vectors may be designed such that the vector will autonomously replicate in the nucleus of the cell. In this case, elements which induce DNA replication may be required in the recombinant vector. Alternatively the recombinant vector may be designed such that the vector and recombinant DNA molecule integrates into the genome of a cell. In this case DNA sequences which favour targeted integration (e.g. by homologous recombination) are desirable. Recombinant vectors may also have DNA coding for genes that may be used as selectable markers in the cloning process.

The recombinant vector may also further comprise a promoter or regulator to control expression of the gene as required.

The DNA molecule may (but not necessarily) be one which becomes incorporated in the DNA of cells of the subject being treated. Undifferentiated cells may be stably transformed leading to the production of genetically modified daughter cells. When this is the case, regulation of expression in the subject may be required e.g. with specific transcription factors, gene activators or more preferably with inducible promoters which transcribe the gene in response to a signal specifically found at a site of epithelial damage. Alternatively, the delivery system may be designed to favour unstable or transient transformation of differentiated cells in the subject being treated. In this instance, regulation of expression may be less important because expression of the DNA molecule will stop when the transformed cells die or stop expressing the protein (ideally when the site of epithelial damage has been effectively regenerated).

The delivery system may provide the DNA molecule to a subject without it being incorporated in a vector. For instance, the DNA molecule may be incorporated within a liposome or virus particle. Alternatively the "naked" DNA molecule may be inserted into a subject's cells by a suitable means e.g. direct endocytotic uptake.

The DNA molecule may be transferred to the cells of a subject to be treated by transfection, infection, microinjection, cell fusion, protoplast fusion or ballistic bombardment. For example, transfer may be by ballistic transfection with coated gold particles, liposomes containing the DNA molecule, viral vectors (e.g. adenovirus) and means of providing direct DNA uptake (e.g. endocytosis) by application of plasmid DNA directly to a site of epithelial damage topically or by injection.

The agent in accordance with the invention expressed from the DNA molecule may be one which directly or indirectly up-regulates TGF-$\beta_3$ expression and/or activity, thereby promoting epithelial regeneration.

Methods of the invention may be put into practice by inducing increased cellular expression of an agent in accordance with the invention, which may then promote epithelial regeneration. Such therapeutic expression of an agent in accordance with the invention may be achieved by increasing naturally occurring expression of the agent (for example the natural expression of a naturally occurring agent such as TGF-$\beta_3$), or by inducing unnatural expression of the agent (e.g. induction of TGF-$\beta_3$ expression by cells that do not naturally express TGF-$\beta_3$) or inducing over-expression of the agent.

Cellular expression of the agent in accordance with the invention, whether natural or unnatural expression, may be by epithelial cells, which may be existing epithelial cells at the edge of the damaged area, or may alternatively be epithelial cells therapeutically introduced into the damaged area (for example cultured endogenous or exogenous epithelial cells). Alternatively cellular expression of the agent in accordance with the invention may be effected by expression of the agent by cells in proximity or contact with the epithelium regeneration of which is to be promoted. For example, in the case where it is wished to promote epidermal regeneration the agent in accordance with the invention may be expressed by cells located in the dermis underlying or surrounding the damaged epithelium.

It will be appreciated that cells that are to be introduced therapeutically to promote epithelial regeneration may be manipulated *ex vivo* such that they express increased levels of an agent in accordance with the invention, and then introduced into the damaged area. As outlined above such cells may themselves be epithelial cells, or may be cells which are situated sufficiently closely to the damaged epithelium that the agent expressed by the cells is able to promote the desired epithelial regeneration. The cells may preferably be cells cultured *ex vivo* for use in the preparation or manufacture of artificial skin or skin substitutes. The cells may more preferably be autologous cells, although it will be appreciated that any suitable cells may be used.

Accordingly, in a eighth aspect of the invention, there is provided a medicament comprising any relevant cell type (for example epithelial, macrophage, monocyte, fibroblast, endothelial or stem cells) induced to express an agent in accordance with the present invention.

The induction of cellular expression of an agent in accordance with the invention may effected by means of external signals influencing the cells, or by means of the incorporation in the cells of nucleic acids causing the expression of the agent in accordance with the fourth to sixth aspects of the invention.

The present invention will further be described in the following non-limiting Examples 1 and 2. Example 1 illustrates the promotion of epithelial regeneration by TGF-$\beta_3$ of full thickness human skin wounds, while Example 2 illustrates that TGF-$\beta_3$ is able to promote epithelial regeneration in partial thickness human skin wounds.

The Examples refer to the accompanying Figures, in which:

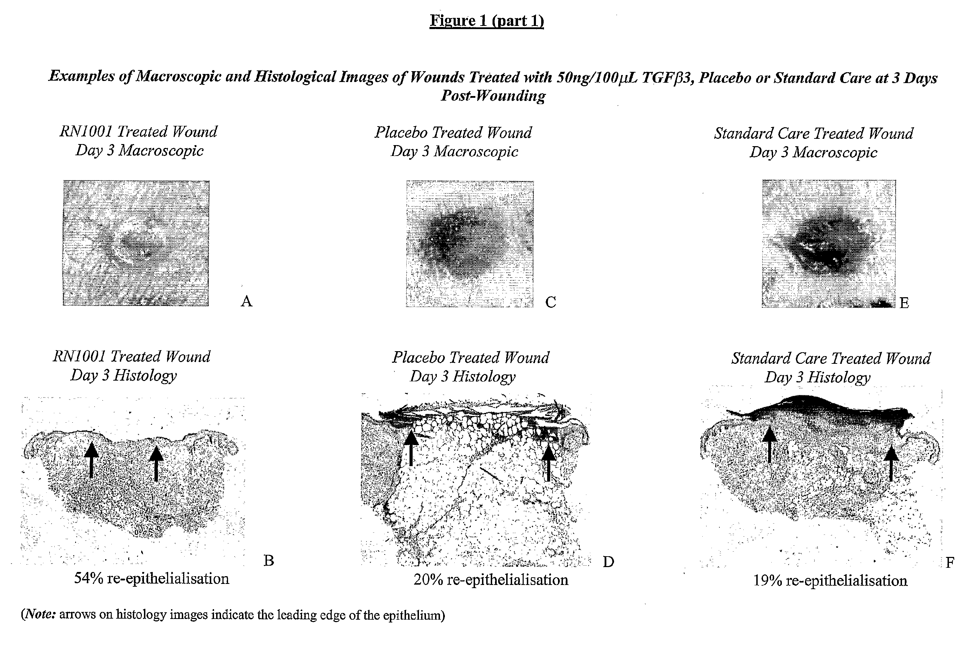

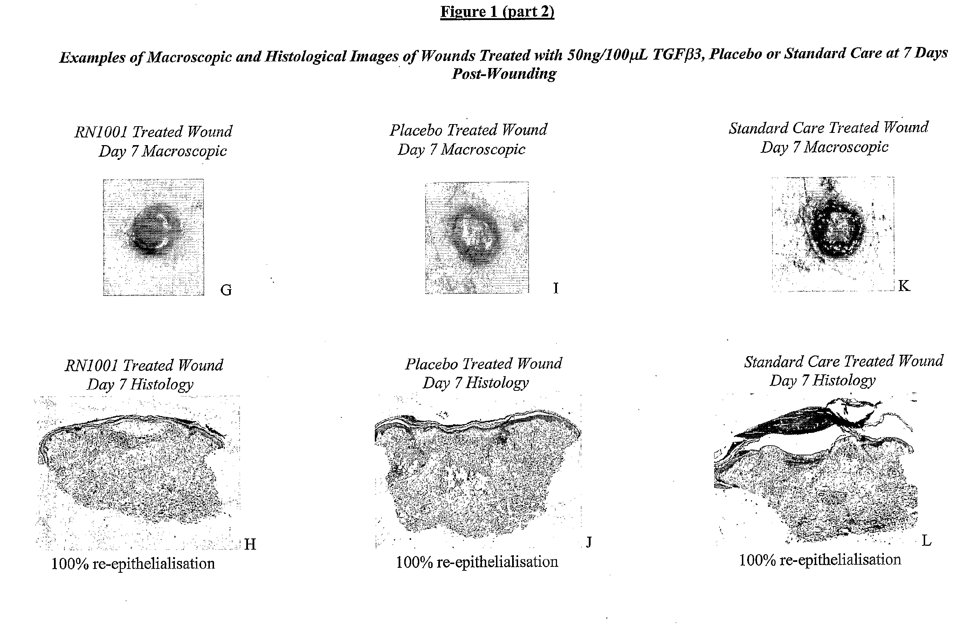

Figure 1, illustrates:

i) photographs, taken at time-points three and seven days post-wounding, illustrating the macroscopic appearance of the full thickness human wounds during epithelial regeneration; and
ii) micrographs from the same time-points illustrating the histology of the damaged sites during epithelial regeneration.

Figure 1 shows examples of macroscopic and histological images of wounds treated with 50ng/100$\mu$L TGF$\beta$3, Placebo or standard care. The results are shown at 3 and 7 days post-wounding.

Specifically, panels A and B respectively show macroscopic and microscopic appearance of TGF-$\beta$3 treated wounds three days post-wounding. Analysis of the histological image reveals 54% re-epithelialisation.

Panels C and D respectively show macroscopic and microscopic appearance of placebo treated wounds three days post-wounding. Analysis of the histological image reveals 20% re-epithelialisation.

Panels E and F respectively show macroscopic and microscopic appearance of standard care treated wounds three days post-wounding. Analysis of the histological image reveals 19% re-epithelialisation.

Panels G and H respectively show macroscopic and microscopic appearance of TGF-β3 treated wounds seven days post-wounding. Analysis of the histological image reveals 100% re-epithelialisation.

Panels I and J respectively show macroscopic and microscopic appearance of placebo treated wounds seven days post-wounding. Analysis of the histological image reveals 100% re-epithelialisation.

Panels K and L respectively show macroscopic and microscopic appearance of standard care treated wounds seven days post-wounding. Analysis of the histological image reveals 100% re-epithelialisation.

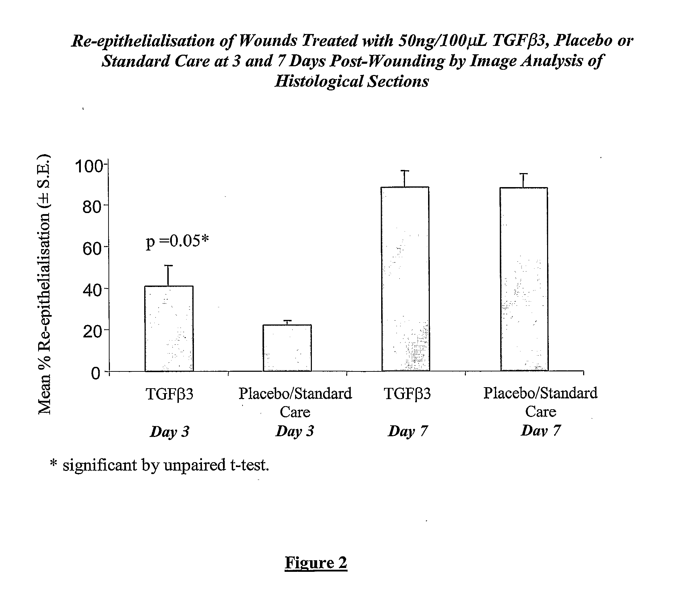

Figure 2, illustrates the comparison between mean percentage re-epithelialisation achieved in TGF-$\beta_3$ treated and untreated/placebo-treated wounds at days three and seven post-injury. Results shown are for re-epithelialisation of wounds treated with 50ng/100μL TGFβ3, Placebo or Standard Care at 3 and 7 Days Post-Wounding. Results were produced by image analysis of histological sections. * Indicates a significant result as assessed by unpaired t-test (p=0.05).

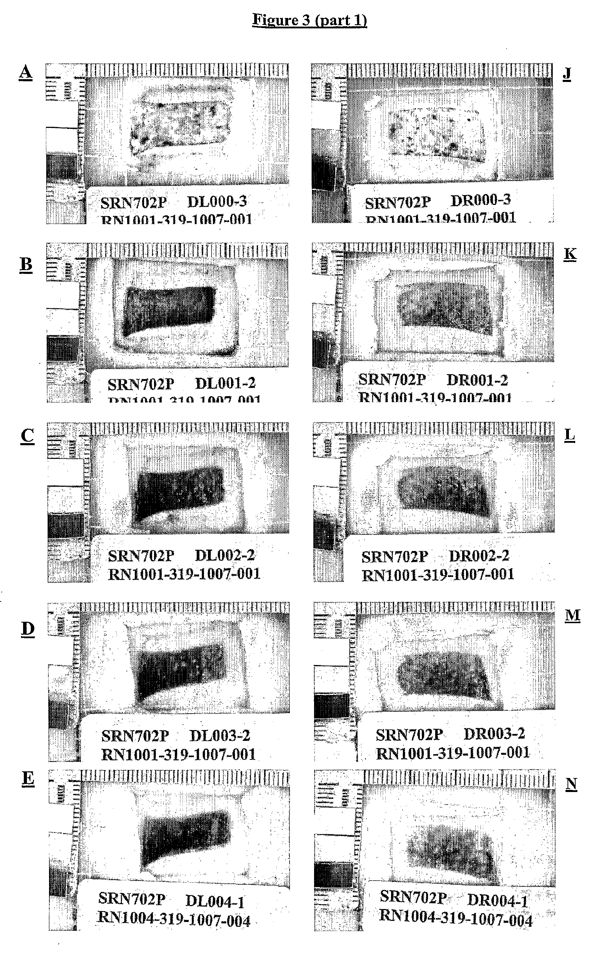

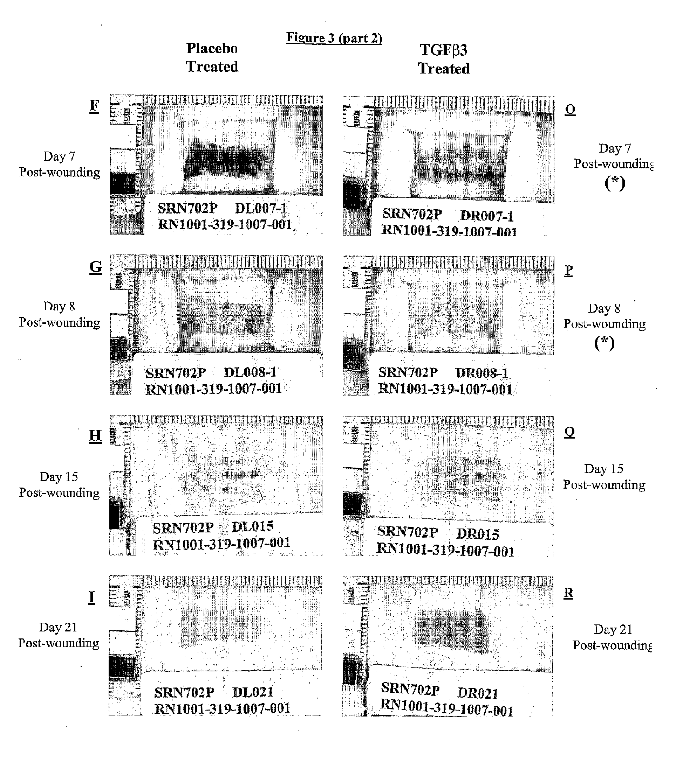

Figure 3, shows photographs illustrating the macroscopic appearance, and thereby progression of epithelial regeneration, in partial thickness skin wounds over 21 days from the time of injury.

Panels A to I show the placebo-treated wounds on days 0, 1, 2, 3, 4, 7, 8, 15 and 21 respectively. In contrast Panels J to R show the TGF-β3-treated wounds on days 0, 1, 2, 3, 4, 7, 8, 15 and 21 respectively

## EXAMPLE 1.

### Effects of TGF-β3 on Re-epithelialisation of Human Full Thickness Wounds

A Phase I study under local Ethical Committee approval was undertaken, comprising a dose escalating first in man (FIM) study in instances of epidermal damage to determine the maximal tolerated dose (MTD) of TGF-$\beta_3$ administered by intra-dermal injection. The study was performed as a double blind, placebo (vehicle) and standard care controlled, randomised, parallel group study to investigate the clinical safety, toleration, systemic pharmacokinetics and local pharmacodynamics of repeated, escalating concentrations of intradermal TGF-$\beta_3$ in Caucasian healthy male volunteer subjects aged 18-45 years (Study and Protocol Reference Number: RN1001-319-1001-001).

A total of seventy two subjects were planned in the study, with planned doses of TGF-$\beta_3$ (and perfectly matching placebos) being 50ng/100μL, 100ng/100μL, 500ng/100μL, 1000ng/100μL, 10μg/100μL and 100μg/100μL.

The data from the study was entered into a Regulatory compliant database that was locked on the 8th December 2003 and the study un-blinded (i.e., randomisation codes released) on the 19th December 2003.

All subjects in the study received two full thickness 3mm punch biopsies to each of their two arms and standard care to all of their wounds. Standard care provided optimal care for moist wound healing in all cases. In addition to this for the pair of wounds in each subject's arms, wounds received TGF-$\beta_3$ versus TGF-$\beta_3$ placebo, TGF-$\beta_3$ versus nothing (denoted as standard care) or TGF-$\beta_3$ placebo versus nothing (standard care). The design of the study was such that comparisons of the effects of treatments on wounds could be made both within and between subjects i.e., individuals acted as their own controls.

The TGF-$\beta_3$ bulk drug substance used in the study was manufactured to GMP and contained acid and alcohol as excipients. This material was serially diluted to provide pre-filled sterile syringes for intra-dermal injection in the study (again manufactured to GMP). The acid and alcohol excipient concentrations varied due to the process of serial dilution to the required TGF-$\beta_3$ injection doses from the bulk drug substance. As the effects of these excipients at different concentrations on both the safety /toleration and effects on healing in man were not known, perfectly matched placebos were also prepared to GMP in the same fashion (i.e., the placebo contained excipients at equivalent concentrations but not TGF-$\beta_3$ protein). This also allowed comparison of the perfectly matched placebos to standard care alone, to demonstrate whether the excipients themselves had any adverse effect on healing.

The study design was such that 9 subjects in each dose group had two punch biopsies made on each arm which had been treated with either TGF-$\beta_3$, placebo or standard care as described above. For each subject's arm the two biopsy sites were marked on the inner aspect and under local anaesthesia TGF-$\beta_3$, placebo or nothing (standard care) was intradermally injected into the site. A 3mm full thickness punch biopsy was then taken from each of the marked sites. On the day after wounding the sites were then treated again, as above, with the same treatment i.e., TGF-$\beta_3$, placebo or nothing (standard care) under local anaesthesia. The wounds were then excised on either day 3 or day 7 post-wounding for histology, to enable analysis of the effects of treatment on wound healing at 3 and 7 days following the initial punch biopsies. The subjects were then followed-up. Safety and toleration data were collected throughout the study.

The wounds were excised, histologically processed into paraffin wax blocks, tissue sections cut and then analysed for re-epithelialisation using image analysis. A total of 36 wounds were generated in this way for each dose group such that a total of 16 wounds were treated with TGF-$\beta_3$, 10 wounds with placebo and 10 wounds with standard care. This resulted in the following wound numbers per treatment at two different time-points post-wounding for histological analysis:

8 TGF-$\beta_3$ treated wounds, 5 placebo treated wounds and 5 standard care treated wounds excised at day 3 post-wounding.
8 TGF-$\beta_3$ treated wounds, 5 placebo treated wounds and 5 standard care treated wounds excised at day 7 post-wounding.

The percentage re-epithelialisation at sites of epidermal damage was calculated according to the following formula:

$$\% \text{ re-epithelialisation} = \frac{(\text{total wound diameter} - \text{non-epithelialised wound diameter})}{\text{total wound diameter}} \times 100$$

### Results.

### Acceleration of Re-epithelialisation in Full Thickness Wounds in Humans by TGF-$\beta_3$.

Progression of epidermal regeneration in full thickness human wounds is illustrated in Figure 1, which includes:

iii) Photographs, taken at time-points three and seven days post-wounding, illustrating the macroscopic appearance of the full thickness wounds during epithelial regeneration; and
iv) micrographs from the same time-points illustrating the histology of the damaged sites during epithelial regeneration.

Analysis of histological sections demonstrated that equivalent re-epithelialisation of wounds occurred in placebo and standard care control treated wounds at both time points post-wounding and as such these groups were combined to compare to TGF-$\beta_3$ treated wounds.
It can be seen from Figure 1 that TGF-$\beta_3$ treatment of full thickness skin wounds is able to promote epithelial regeneration, leading to reconstitution of the epidermis earlier than is the case in untreated or placebo-treated wounds.
The results of Example 1 are shown in Figure 2, which compares mean percentage re-epithelialisation in TGF-$\beta_3$ treated and untreated/placebo-treated wounds at days three and seven post-injury.
Figure 2 clearly illustrates that treatment with TGF-$\beta_3$ at a concentration of 50ng/100$\mu$L significantly accelerated wound re-epithelialisation when compared to re-epithelialisation occurring in control wounds. The promotion of epithelial regeneration in drug treated versus control wounds was also observed in individuals receiving TGF-$\beta_3$ at up to (500ng/100$\mu$L).

### EXAMPLE 2.

### Effects of TGF-$\beta_3$ on Re-epithelialisation of Human Partial Thickness Wounds / Skin Graft Donor Sites

A pilot study (non-blinded) was carried out under local Ethical Committee approval, to investigate the effects of intra-dermal and topical applications of TGF-$\beta_3$ when applied to split thickness skin graft donor sites in two healthy Caucasian male human volunteer subjects aged 18-45 years. The concentration of TGF-$\beta_3$ used was 50ng/100$\mu$L, and the rate of epithelial regeneration achieved was compared to a perfectly matched TGF-$\beta_3$ placebo at the equivalent concentration. Both the TGF-$\beta_3$ and placebo were prepared to GMP as described above.
The donor sites were firstly identified and marked out on each side of the midline on the lower back, each measuring 1.5 by 2cm, and then infiltrated with local anaesthetic containing 1 in 200,000 adrenalin. Each site then received an intradermal injection such that one side received TGF-$\beta_3$ at a dose of 50ng/cm$^2$ and the other side received perfectly matched placebo. Subjects then rested in the prone position for 30 minutes before an approximately 0.55mm thick split thickness skin graft was harvested from each of the marked sites, with haemostasis being achieved with gentle pressure. Immediately following graft harvest each donor site was given a topical application of either TGF-$\beta_3$ or placebo the wounds were then dressed and the subject discharged. Subjects returned the next day and had a further topical application of either TGF-$\beta_3$ or placebo such that at all stages each wound received the same treatment, i.e., three applications of TGF-$\beta_3$ or three applications of placebo. Subjects were then reviewed on a daily basis for a two week period and then at 21 days, with subsequent follow-up.

**Results:**

**Acceleration of Re-epithelialisation of Partial Thickness Wounds / Skin Graft Donor Sites in Humans by TGF-$\beta_3$.**

Macroscopic analysis, assessed using photographs of the damaged sites, demonstrated that TGF-$\beta_3$ markedly accelerated the promotion of epidermal regeneration, increasing the rate of re-epithelialisation at partial thickness skin graft donor sites, as compared to the rate occurring in placebo treated controls, up to day 8 post-wounding.

Figure 3, which shows photographs illustrating the macroscopic appearance, and thereby progression of epithelial regeneration, in partial thickness skin wounds over 21 days from the time of injury. Photographs demonstrating the promotion of epithelial regeneration are marked with an asterisk (*) in Figure 3.

It can be observed that at days 15 and 21 post-wounding the difference between TGF-$\beta_3$ treated and untreated/ placebo-treated wound is less clear macroscopically. This effect is explained by the fact that TGF-$\beta_3$ treated sites are totally re-epithelialised on or before day 8, while the placebo treated sites only reach the same degree of re-epithelialisation at around day 15.

**CONCLUSIONS (EXAMPLES 1 & 2).**

The results set out above surprisingly demonstrate that a specific member of the TGF-$\beta$ family, TGF-$\beta_3$, is able to promote epithelial regeneration. This finding is in direct contrast to that which the skilled person would expect in the light of the prior art, since previous reports have suggested that members of the TGF-$\beta$ family inhibit regeneration of the epidermis in skin wounds, with the TGF-$\beta_3$ isoform being the most potent inhibitor of this process. The promotion of epithelial regeneration brought about by treatment with TGF-$\beta_3$ is achieved in both full thickness wounds and partial thickness wounds (which may, for instance, serve as skin graft donor sites). The therapeutic effects in humans are observed in TGF-$\beta_3$ treatment utilising doses up to 500ng/10$\mu$L of the active agent.

**APPENDIX**

**Formulations.**

Details of the formulations used in clinical studies to establish the ability of agents having TGF-$\beta_3$ activity to promote epithelial regeneration are provided under the following headings.

TGF-$\beta_3$ drug substance used in the studies was supplied at a concentration of 9.1mg/mL in 20mM acetic acid and 20% isopropyl alcohol. This material was serially diluted to produce syringes containing TGF-$\beta_3$ at the concentrations stated in the exemplar data. It will be appreciated by the skilled person that, irrespective of the further diluents used to produce the final solutions, trace levels of acetic acid and isopropyl alcohol will be carried through by serial dilution.

*A1. Formulations used in treatment of punch biopsy (full thickness dermal wounds)*

For the punch biopsy / full thickness wound studies the drug substance was diluted in PBS containing 5% w/v mannitol, and adjusted to pH 3.8 using acetic acid. This formulation was found to be effective in the promotion of epithelial regeneration.

By way of an alternative to the mannitol-based formulation described above, a maltose-based formulation (described more fully below) was also prepared. This formulation of agents in accordance with the invention in combination with maltose was surprisingly found to dramatically reduce pain associated with injection of the composition. Studies undertaken by the inventors demonstrated clinically that the pain associated with injection of the mannitol-based formulation in the punch biopsy study was alleviated when agents in accordance with the invention were formulated in the presence of sugars, and in particular in the presence of isotonic concentrations of maltose.

*In vitro* analysis of the efficacy of the maltose-based and mannitol-based formulations revealed a further surprising advantage of the compositions comprising active agents in accordance with the invention in combination with maltose. Investigation using an ELISA assay and a Mink Lung Epithelial Cell assay to compare the PBS/mannitol formulation with the 0.25M maltose formulation demonstrates that the maltose formulation results in approximately a 4-fold increase in TGF-$\beta_3$ activity.

*A2. Formulations used in treatment of split thickness skin graft donor sites:*

For this study TGF-$\beta_3$ was formulated in 0.25M maltose (i.e., 90g maltose per litre of water for injection;

equivalent to 9% (w/v) maltose in water for injection).

This formulation was applied both intradermally and topically to promote epithelial regeneration.

## REFERENCES:

Tomlinson A, Ferguson MW. Wound healing: a model of dermal wound repair. Methods Mol Biol 2003; 225: 249-260.

Davidson JM, Nanney LB, Broadley KN, Whitsett JS, Aquino AM, Beccaro M, Rastrelli A. Hyaluronate derivatives and their application to wound healing: preliminary observations. Clin Mater. 1991; 8(1-2):171-7.

Paddock HN, Schultz GS, Mast BA. Methods in reepithelialization. A porcine model ofpartial-thickness wounds. Methods Mol Med. 2003; 78:17-36.

Rakel BA, Bermel MA, Abbott LI, Baumler SK, Burger MR, Dawson CJ, Heinle JA, Ocheltree IM. Split-thickness skin graft donor site care: a quantitative synthesis of the research. Appl Nurs Res. 1998 Nov; 11(4);174-82.

The present application and invention further includes the subject matter of the following numbered paragraphs which correspond to the claims as filed of WO2007/007098 from which this application is derived.

Paragraphs:

1. The use of an agent having TGF-$\beta_3$ activity in the manufacture of a medicament for the promotion of epithelial regeneration.

2. The use according to paragraph 1, wherein the agent is TGF-$\beta_3$.

3. The use according to paragraph 1, wherein the agents is a fragment, derivative or variant of TGF-$\beta_3$.

4. The use according to paragraph 1, wherein the agent is a substance able to promote and/or mimic the biological activity of TGF-$\beta_3$.

5. The use according to any one of paragraphs 1 to 4, wherein the epithelial regeneration is at a site of an acute wound.

6. The use according to any one of paragraphs 1 to 5, wherein the epithelial regeneration is of a young and/or healthy patient.

7. The use according to any one of paragraphs 1 to 6, wherein the epithelium regeneration of which is to be promoted is a stratified squamous epithelium.

8. The use according to paragraph 7, wherein the epithelium is the epidermis.

9. The use according to paragraph 7, wherein the epithelium is the corneal epithelium.

10. The use according to any one of paragraphs 1 to 6, wherein the epithelium is a respiratory epithelium.

11. The use according to any one of paragraphs 1 to 6, wherein the epithelium is the lining epithelium of the abdomen, thoracic or pelvic cavities.

12. The use according to any preceding paragraph, wherein the medicament is for the promotion of epithelial regeneration after injury.

13. The use according to paragraph 10, wherein the medicament is for the promotion of epithelial regeneration after surgery.

14. The use according to paragraph 12, wherein the surgery comprises epithelial grafting.

15. The use according to paragraph 14, wherein the surgery comprises:

i) epithelial graft removal;

ii) optionally meshing of the epithelial graft; and

iii) application of the graft to a recipient site.

16. The use according to paragraph 14, wherein the medicament is for administration to the graft donor site.

17. The use according to paragraph 14, wherein the medicament is for administration to the graft recipient site.

18. The use according to paragraph 14, wherein the medicament is for administration to the graft.

19. The use according to any of paragraphs 12 to 18, wherein the surgery comprises skin grafting.

20. The use according to paragraph 19, wherein the skin graft is a full thickness skin graft.

21. The use according to paragraph 19, wherein the skin graft is a partial thickness skin graft.

22. The use according to any preceding paragraph, wherein the medicament is for the promotion of epithelial regeneration after burn injury.

23. The use according to any preceding paragraph, wherein the medicament is for topical application.

24. The use according to any one of paragraphs 1 to 23, wherein the medicament is for local injection.

25. The use according to any one of paragraphs 1 to 23, wherein the medicament is a cream or ointment.

26. The use according to any preceding paragraph, wherein the promotion of epithelial regeneration is prophylactic promotion.

27. The use According to any of paragraphs 1 to 5, or 7 to 26, for the preparation of a medicament to promote impaired, inhibited, retarded, or otherwise defective epithelial regeneration.

28. The use According to any one of paragraphs 1 to 26 for the preparation of a medicament to accelerate normal epithelial regeneration.

29. The use according to any of the preceding paragraphs for the preparation of a medicament to promote epithelial regeneration in the aged.

30. The use according to any preceding paragraph, wherein the agent is formulated in the presence of maltose.

31. The use according to paragraph 30, wherein the maltose is present at a concentration between 0.1M and 0.4M maltose.

32. The use according to paragraph 30, wherein the maltose is present at a concentration of 0.25M maltose.

## Claims

1. An agent having TGF-$\beta_3$ activity for use in the promotion of epithelial regeneration.

2. An agent according to claim 1, selected from the group consisting of TGF-$\beta_3$, a fragment, derivative or variant of TGF-$\beta_3$, a substance able to promote and/or mimic the biological activity of TGF-$\beta_3$.

3. An agent according to claim 1 or claim 2, for use in a formulation further comprising a sugar.

4. An agent according to claim 3, wherein the sugar is selected from the group consisting of: monosaccharides and disaccharides, such as maltose, mannose, sucrose, glucose, or trehalose.

5. An agent according to any preceding claim for local injection.

6. An agent according to any preceding claim, for use in an injectable solution containing between 50ng/100$\mu$l and 500ng/100$\mu$l of the agent.

7. The use according to any one of claims 1 to 3, wherein the medicament is a cream or ointment.

8. An agent according to any preceding claim, for promoting epithelial regeneration of the epidermis.

9. An agent according to any preceding claim, for use in the promotion of epithelial regeneration after injury.

10. An agent according to claim 9, for use in the promotion of epithelial regeneration after surgery, such as epithelial grafting, for example by administration to the graft donor site, or by administration to the graft recipient site.

11. An agent according to any preceding claim, for use in prophylactic promotion of epithelial regeneration.

12. An agent according to any preceding claim, for use to accelerate normal epithelial regeneration.

13. An agent according to any preceding claim, formulated as a lyophilised or non-lyophilised powder for reconstitution.

**14.** A method of preparing a therapeutic formulation suitable for localised parenteral administration, for use in the promotion of epithelial regeneration, the method comprising mixing an agent having TGF-β3 activity with a at least one physiologically acceptable carrier, excipient or stabilizer, and lyophilising the mixture to produce a powder for reconstitution.

**15.** A therapeutically effective amount of an agent having TGF-$\beta_3$ activity for use preparing an epithelial graft donor site for re-harvesting.

**16.** A delivery system for use in a gene therapy technique, said delivery system comprising a DNA molecule encoding for an agent having TGF-$\beta_3$ activity, said DNA molecule being capable of being transcribed to lead to the expression of the chosen agent,

**17.** A delivery system according to claim 16, for use in the promotion of epithelial regeneration.

**18.** A relevant cell type, such as epithelial, macrophage, monocyte, fibroblast, endothelial or stem cells, induced to express an agent having TGF-$\beta_3$ activity, for use as a medicament.

# Figure 1 (part 1)

*Examples of Macroscopic and Histological Images of Wounds Treated with 50ng/100μL TGFβ3, Placebo or Standard Care at 3 Days Post-Wounding*

*RN1001 Treated Wound*
*Day 3 Macroscopic*

*Placebo Treated Wound*
*Day 3 Macroscopic*

*Standard Care Treated Wound*
*Day 3 Macroscopic*

A

C

E

*RN1001 Treated Wound*
*Day 3 Histology*

*Placebo Treated Wound*
*Day 3 Histology*

*Standard Care Treated Wound*
*Day 3 Histology*

B

D

F

54% re-epithelialisation

20% re-epithelialisation

19% re-epithelialisation

(*Note:* arrows on histology images indicate the leading edge of the epithelium)

**Figure 1 (part 2)**

*Examples of Macroscopic and Histological Images of Wounds Treated with 50ng/100μL TGFβ3, Placebo or Standard Care at 7 Days Post-Wounding*

*Standard Care Treated Wound Day 7 Macroscopic* — K

*Standard Care Treated Wound Day 7 Histology* — L
100% re-epithelialisation

*Placebo Treated Wound Day 7 Macroscopic* — I

*Placebo Treated Wound Day 7 Histology* — J
100% re-epithelialisation

*RN1001 Treated Wound Day 7 Macroscopic* — G

*RN1001 Treated Wound Day 7 Histology* — H
100% re-epithelialisation

*Re-epithelialisation of Wounds Treated with 50ng/100μL TGFβ3, Placebo or Standard Care at 3 and 7 Days Post-Wounding by Image Analysis of Histological Sections*

\* significant by unpaired t-test.

**Figure 2**

## Figure 3 (part 1)

**A** SRN702P  DL000-3  RN1001-319-1007-001

**J** SRN702P  DR000-3  RN1001-319-1007-001

**B** SRN702P  DL001-2  RN1001-319-1007-001

**K** SRN702P  DR001-2  RN1001-319-1007-001

**C** SRN702P  DL002-2  RN1001-319-1007-001

**L** SRN702P  DR002-2  RN1001-319-1007-001

**D** SRN702P  DL003-2  RN1001-319-1007-001

**M** SRN702P  DR003-2  RN1001-319-1007-001

**E** SRN702P  DL004-1  RN1004-319-1007-004

**N** SRN702P  DR004-1  RN1004-319-1007-004

## Figure 3 (part 2)

**Placebo Treated**  **TGFβ3 Treated**

F — Day 7 Post-wounding
SRN702P DL007-1 RN1001-319-1007-001

O — Day 7 Post-wounding (*)
SRN702P DR007-1 RN1001-319-1007-001

G — Day 8 Post-wounding
SRN702P DL008-1 RN1001-319-1007-001

P — Day 8 Post-wounding (*)
SRN702P DR008-1 RN1001-319-1007-001

H — Day 15 Post-wounding
SRN702P DL015 RN1001-319-1007-001

Q — Day 15 Post-wounding
SRN702P DR015 RN1001-319-1007-001

I — Day 21 Post-wounding
SRN702P DL021 RN1001-319-1007-001

R — Day 21 Post-wounding
SRN702P DR021 RN1001-319-1007-001

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0684260 A
- WO 2007007098 A

**Non-patent literature cited in the description**

- **TOMLINSON A ; FERGUSON MW.** Wound healing: a model of dermal wound repair. *Methods Mol Biol,* 2003, vol. 225, 249-260
- **DAVIDSON JM ; NANNEY LB ; BROADLEY KN ; WHITSETT JS ; AQUINO AM ; BECCARO M ; RASTRELLI A.** Hyaluronate derivatives and their application to wound healing: preliminary observations. *Clin Mater.,* 1991, vol. 8 (1-2), 171-7
- **PADDOCK HN ; SCHULTZ GS ; MAST BA.** Methods in reepithelialization. A porcine model of partial-thickness wounds. *Methods Mol Med.,* 2003, vol. 78, 17-36
- **RAKEL BA ; BERMEL MA ; ABBOTT LI ; BAUMLER SK ; BURGER MR ; DAWSON CJ ; HEINLE JA ; OCHELTREE IM.** Split-thickness skin graft donor site care: a quantitative synthesis of the research. *Appl Nurs Res.,* November 1998, vol. 11 (4), 174-82